# EUROPÄISCHE PATENTSCHRIFT

(11) **EP 2 367 783 B1**
(45) Veröffentlichungstag und Bekanntmachung des Hinweises auf die Patenterteilung: **09.01.2013**
(21) Anmeldenummer: 09752383.1
(22) Anmeldetag: 18.11.2009
(51) Int. Cl.: C07C 263/10, C07C 265/14, C07C 209/90

(54) **VERFAHREN ZUR HERSTELLUNG EINES ISOCYANATS**
METHOD FOR PRODUCING AN ISOCYANATE
PROCÉDÉ DE PRODUCTION D'UN ISOCYANATE

(30) Priorität: 19.11.2008 EP 08169399
(43) Veröffentlichungstag der Anmeldung: 28.09.2011
(73) Patentinhaber: BASF SE, 67056 Ludwigshafen (DE)
(72) Erfinder: BREUNINGER, Daniel, 67240 Bobenheim-Roxheim (DE); ADAM, Johannes, 01109 Dresden (DE); SCHELLING, Heiner, 67281 Kirchheim (DE); STROEFER, Eckhard, 68163 Mannheim (DE); KRÄMER, Markus, 01471 Radeburg (DE); EIERMANN, Matthias, 67117 Limburgerhof (DE); THIELE, Kai, B-2040 Antwerpen 4 (BE); ZOELLINGER, Michael, 01099 Dresden (DE); PINKOS, Rolf, 67098 Bad Dürkheim (DE)
(86) Internationale Anmeldenummer: PCT/EP2009/065373
(87) Internationale Veröffentlichungsnummer: WO 2010/057909

(56) Entgegenhaltungen:
- EP-A1- 0 446 781
- EP-A2- 0 546 400
- EP-A2- 0 866 057

## Beschreibung

Die vorliegende Erfindung betrifft ein Verfahren zur Herstellung eines Isocyanats, umfassend Hydrieren eines ein Amin enthaltenden Gemischs (Gi) in Anwesenheit eines Kupfer enthaltenden Hydrierkatalysators unter Erhalt eines das Amin enthaltenden Gemischs (Gii), und Umsetzen des Gemisches (Gii) mit Phosgen unter Erhalt eines das Isocyanat enthaltenden Gemischs (Giii). Weiterhin betrifft die vorliegende Erfindung das Isocyanat, herstellbar nach dem erfindungsgemäßen Verfahren.

Isocyanate sind sehr wichtige Rohstoffe für die Herstellung von Polyurethanen. Technisch wichtig sind insbesondere die Di- und Polyisocyanate der Diphenylmethan-Reihe (MDI). Der allgemeine Begriff "MDI", wie er im Rahmen der vorliegenden Erfindung verwendet wird, bezeichnet Gemische aus Diisocyanaten und Polyisocyanaten der Diphenylmethan-Reihe.

Unter einem Diisocyanat bzw. einem Polyisocyanat der Diphenylmethan-Reihe wird ein Isocyanat des folgenden Typs verstanden: 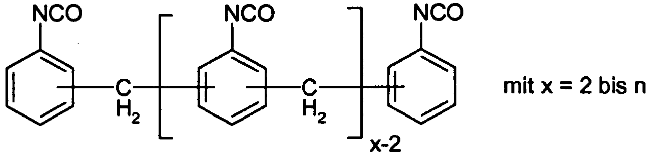 wobei n eine Ganzzahl von größer 1, beispielsweise 2, 3, 4, 5, 6, 7, 8, 9, 10 oder größer als 10 ist. Handelt es sich um ein Diisocyanat, so ist n 2. Handelt es sich um ein Polyisocyanat, so ist n größer als 2.

Es ist bekannt, dass MDI durch Phosgenierung von MDA hergestellt werden kann. Der allgemeine Begriff "MDA", wie er im Rahmen der vorliegenden Erfindung verwendet wird, bezeichnet Gemische aus Diaminen und Polyaminen der Diphenylmethan-Reihe.

Unter einem Diamin bzw. einem Polyamin der Diphenylmethan-Reihe wird ein Amin des folgenden Typs verstanden: 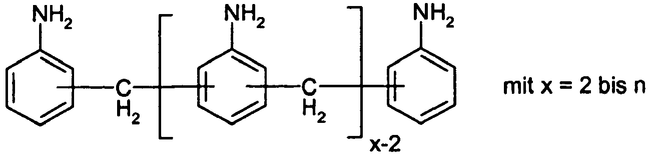 wobei n eine Ganzzahl von größer 1, beispielsweise 2, 3, 4, 5, 6, 7, 8, 9, 10 oder größer als 10 ist. Handelt es sich um ein Diamin, so ist n 2. Handelt es sich um ein Polyamin, so ist n größer als 2.

Typischerweise erfolgt die Synthese von MDI in einem zweistufigen Prozess, wobei zunächst Anilin mit Formaldehyd zu MDA umgesetzt wird und das MDA anschließend in einem zweiten Schritt mit Phosgen umgesetzt wird. Die Phosgenierung von MDA ist dem Fachmann bekannt und beispielsweise in H. Ullrich "Chemistry and Technology of Isocyanates", John Wiley, 1996 beschrieben.

Die Phosgenierung von MDA hat den Nachteil, dass bei der Phosgenierung höchst unerwünschte Verfärbungen auftreten, die durch Reaktion von im MDA enthaltenen Verunreinigungen mit Phosgen entstehen und die bei der Weiterverarbeitung des MDI zu den Polyurethanen erhalten bleiben. Ein weiteres, für die Qualität des MDI nachteiliges Problem ist die Verunreinigung des MDI durch chlorhaltige Komponenten.

Zur empirischen Farbaufhellung von MDI sind zahlreiche Methoden bekannt. Um die genannten unerwünschten Verfärbungen zu reduzieren, schlagen beispielsweise die DE-A 33 29 124 und die US 3,479,384 vor, das MDI, welches aus der Phosgenierung erhalten wird, durch extraktive Reinigungsverfahren aufzuarbeiten.

Die US 4,189,354 schlägt die Verwendung zusätzlicher Destillationsschritte zur Reinigung der MDI-Mischungen vor, wodurch geringere Mengen an chlorhaltigen Komponenten und dadurch geringere Mengen an hydrolysierbarem Chlor als Verunreinigung im MDI zurückbleiben sollen. Durch die geringere Menge an chlorhaltigen Komponenten soll die Qualität der aus dem MDI hergestellten Polyurethane verbessert werden.

Die EP-A 0 816 333 beschreibt ein Verfahren zur Herstellung von MDI, wobei eine Lösung, enthaltend mindestens ein Isocyanat, nach der Phosgenierung und vor der vollständigen Abtrennung des Lösungsmittels einer Behandlung mit Wasserstoff in Gegenwart von Katalysatoren, enthaltend Übergangsmetallverbindungen der Gruppe I, VII und VIII, unterzogen wird. Explizit werden nur Katalysatoren, enthaltend Edelmetalle, insbesondere Palladium, Platin, Rhodium und Ruthenium, offenbart.

Ein alternatives Verfahren zur Aufhellung von MDI wird in der US 4,465,639 beschrieben. Hier wird eine Aufhellung von MDI erreicht, indem die Reaktionslösung direkt nach der Phosgenierung von MDA und vor dem Entfernen des überschüssigen Phosgens mit Wasser behandelt wird. Der aufwändige Aufarbeitungsschritt der obigen Verfahren entfällt zwar, doch wird auch hier die Verfärbung nicht vermieden und muss durch eine Nachbehandlung des MDI mit Wasser reduziert werden. Diese Wasserbehandlung führt wiederum zu Korrosionsproblemen aufgrund des entstehenden Chlorwasserstoffs.

Die oben beschriebenen Verfahren haben den Nachteil, dass die bei der Phosgenierung entstehenden Verfärbungen, also das Entstehen der die Verfärbung hervorrufenden Bestandteile, nicht vermieden werden, sondern die einmal entstandenen Verfärbungen oder zumindest ein Teil dieser entstandenen Verfärbungen erst nachträglich entfernt werden. Für diese Entfernung können aufwändige zusätzliche Aufarbeitungs- und Entsorgungsschritte notwendig sein.

Gemäß EP-A 0 866 057 wird eine Qualitätsverbesserung von MDI erreicht, indem nicht die bereits entstandenen Verfärbungen, die im MDI enthalten sind, entfernt werden, sondern bereits die Vorstufe MDA entsprechend behandelt wird. Die Behandlung besteht darin, das MDA vor seiner Umsetzung mit festen anorganischen Lewissäuren und/oder Brönstedtsäuren in Kontakt zu bringen.

Anstelle der Behandlung mit Lewissäuren schlägt die EP-A 0 446 781 eine Behandlung von MDA durch Hydrierung des MDA mit Wasserstoff in Gegenwart eines Hydrierkatalysators vor. Als Hydrierkatalysatoren werden Katalysatoren beschrieben, die Platinmetalle sowie weiterhin Nickel, Metalle oder Oxide von Wolfram und Molybdän oder Gemische verschiedener Metalle wie beispielsweise Nickel und Molybdän enthalten. Das hydrierte MDA wird dann mit Phosgen zu MDI umgesetzt, welches eine verbesserte Farbzahl aufweisen soll. Kupferhaltige Katalysatoren werden nicht beschrieben.

Auch die EP-A 0 546 400 und die US 5,889,070 beschreiben Verfahren zur Verbesserung der Farbe von MDA durch Hydrieren des MDA in Anwesenheit eines Metallkatalysators. Als Metallkatalysatoren werden Katalysatoren der Gruppe 8A wie beispielsweise Nickel, Palladium, Platin, Kobalt, Ruthenium und Rhodium genannt.

Die im Stand der Technik beschriebenen Verfahren, die zusätzliche Aufreinigungsschritte umfassen, sind wie oben beschrieben sehr aufwändig und unwirtschaftlich. Die im Stand der Technik beschriebenen Verfahren, bei denen zusätzliche Aufreinigungsschritte entfallen, haben wiederum den Nachteil, dass zumeist sehr teure Katalysatoren zur Hydrierung verwendet werden und dass diese Katalysatoren oft Nebenreaktionen begünstigen, die im MDA bzw. im daraus hergestellten MDI zu chlorhaltigen Nebenprodukten führen. Dies führt zu einer nachteiligen Erhöhung der Chlorwerte im MDI.

Demnach besteht weiterhin der Bedarf nach neuen Verfahren zur Herstellung von Isocyanaten, insbesondere zur Herstellung von Isocyanaten der Diphenylmethan-Reihe, mit vorteilhafter Qualität. Unter dem Begriff "Isocyanaten mit vorteilhafte Qualität" werden im Rahmen der vorliegenden Erfindung Isocyanate verstanden, die geringe Mengen an farbgebenden Substanzen sowie einen geringen Gehalt an chlorhaltigen Komponenten aufweisen.

Eine der der vorliegenden Erfindung zugrunde liegenden Aufgaben war es demnach, ein neues Verfahren zur Herstellung eines Isocyanats bereitzustellen, wobei das Isocyanat nur geringe Mengen an farbgebenden Komponenten sowie einen geringen Gehalt an Chlor bzw. chlorhaltigen Komponenten aufweist.

Diese Aufgabe konnte überraschenderweise durch ein Verfahren zur Herstellung eines Isocyanats gelöst werden, umfassend
(i) Hydrieren eines ein Amin enthaltenden Gemischs (Gi) in Anwesenheit eines Kupfer enthaltenden Hydrierkatalysators unter Erhalt eines das Amin enthaltenden Gemischs (Gii),
(ii) Umsetzen des Gemischs (Gii) mit Phosgen unter Erhalt eines das Isocyanat enthaltenden Gemischs (Giii).

Überraschenderweise wird durch die Verwendung von Kupfer enthaltenden Hydrierkatalysatoren in (i) ein das Isocyanat enthaltendes Gemisch erhalten, welches sowohl geringere Mengen an farbgebenden Substanzen enthält als auch geringere Chlorwerte aufweist als ein Isocyanat, das mit anderen Metallkatalysatoren hergestellt wird.

Weiterhin betrifft die vorliegende Erfindung das Isocyanat, herstellbar nach diesem Verfahren.

Die Formulierung "herstellbar nach einem Verfahren", wie sie im Rahmen der vorliegenden Anmeldung verwendet wird, umfasst sowohl die Formulierung "herstellbar durch ein Verfahren" als auch die Formulierung "hergestellt durch ein Verfahren".

Der Begriff "Amin", wie er im Rahmen der Erfindung verwendet wird, bezeichnet mindestens ein Polyamin oder mindestens ein Diamin oder ein Gemisch aus mindestens einem Polyamin und mindestens einem Diamin. Demnach ist das "Amin" beispielsweise ein Diamin, oder ein Polyamin, oder ein Gemisch aus einem Diamin und einem Polyamin, oder ein Gemisch aus mindestens zwei Diaminen, beispielsweise aus 2, 3, 4, 5 oder 6 Diaminen, oder ein Gemisch aus mindestens zwei Polyaminen, beispielsweise 2, 3, 4, 5 oder 6 Polyaminen, oder ein Gemisch aus einem Diamin und aus mindestens zwei Polyaminen, oder ein Gemisch aus mindestens zwei Diaminen und aus einem Polyamin, oder ein Gemisch aus mindestens zwei Diaminen und mindestens zwei Polyaminen.

Enthält das Amin mindestens ein Diamin, so ist das mindestens eine Diamin bevorzugt ausgewählt aus der Gruppe bestehend aus Hexamethylendiaminen, Isophorondiamin, Cyclohexyldiaminen, Phenyldiaminen Toluyldiaminen, Naphtylendiaminen, Diphenylmethandiaminen, Dicyclohexylmethandiamin und aus Gemischen aus zwei oder mehr dieser Verbindungen.

Weiter bevorzugt ist das mindestens eine Diamin ausgewählt aus der Gruppe bestehend aus 1,6-Hexamethylendiamin, Isophorondiamin, Cyclohexyldiaminen, 2,4-Toluylendiamin, 2,6-Toluylendiamin, 1,5-Naphthylendiamin, 2,2'-Diphenylmethandiamin, 2,4'-Diphenylmethandiamin, 4,4'-Diphenylmethandiamin und Gemischen aus zwei oder mehr dieser Verbindungen.

Enthält das Amin zwei oder mehr Diamine, so handelt es sich bei den zwei oder mehr Diaminen bevorzugt um zueinander isomere Diamine.

Besonders bevorzugt ist das mindestens eine Diamin ein Diamin der Diphenylmethan-Reihe, insbesondere ein Diamin ausgewählt aus der Gruppe bestehend aus 2,2'-Diphenylmethandiamin, 2,4'-Diphenylmethandiamin und 4,4'-Diphenylmethandiamin und Gemischen aus zwei oder mehr dieser Verbindungen.

Enthält das Amin mindestens ein Polyamin, so handelt es sich bei dem mindestens einen Polyamin bevorzugt um ein mehrkemiges oder höhermolekulares Derivat von einem der oben genannten Diamine oder aus Gemischen aus zwei oder mehr dieser Verbindungen. Besonders bevorzugt ist das mindestens eine Polyamin ein Polyamin der Diphenylmethan-Reihe.

Gemäß einer bevorzugten Ausführungsform ist das Amin ausgewählt aus der Gruppe bestehend aus einem Diamin der Diphenylmethan-Reihe, einem Gemisch aus mindestens zwei Diaminen der Diphenylmethan-Reihe, einem Polyamin der Diphenylmethan-Reihe, einem Gemisch aus mindestens zwei Polyaminen der Diphenylmethan-Reihe, und einem Gemisch aus mindestens einem Diamin der Diphenylmethan-Reihe mit mindestens einem Polyamin der Diphenylmethan-Reihe.

Weiter bevorzugt handelt es sich bei dem Amin um ein Gemisch aus mindestens zwei Diaminen der Diphenylmethan-Reihe und mindestens zwei Polyaminen der Diphenylmethan-Reihe.

Demnach beschreibt die vorliegende Erfindung auch ein Verfahren, wie oben beschrieben, wobei das Amin ein Gemisch aus mindestens zwei Diaminen der Diphenylmethan-Reihe und mindestens zwei Polyaminen der Diphenylmethan-Reihe ist. Weiterhin beschreibt die vorliegende Erfindung das Polyisocyanat, herstellbar durch dieses Verfahren.

Besonders bevorzugt ist das Amin ein Gemisch, mindestens enthaltend die Isomere 2,2'-Diphenylmethandiamin, 2,4'-Diphenylmethandiamin und 4,4'-Diphenylmethandiamin, die im Rahmen der Erfindung auch als 2-Kem-MDA oder als Monomer-MDA oder MMDA bezeichnet werden, und weiterhin enthaltend mindestens ein Polyamin der Diphenylmethan-Reihe. Das mindestens eine Polyamin ist beispielsweise ein 3-Kem-MDA, also ein Amin der folgenden Formel, wobei n gleich 3 ist, oder ein höheres Oligomer, also ein Amin der folgenden Formel, wobei n größer 3 ist.

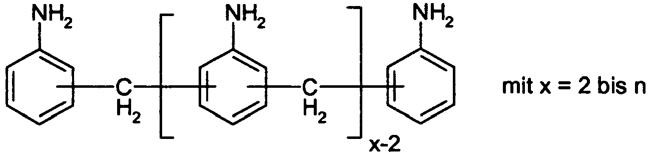

Der Begriff 3-Kem-MDA schließt alle möglichen isomeren Formen der untenstehenden Formel mit n gleich 3 ein. Ebenso sind durch den Begriff Oligomer alle isomeren Formen der einzelnen höherkemigen Oligomere umfasst.

Demnach beschreibt die vorliegende Erfindung auch ein Verfahren wie oben beschrieben, wobei das Amin ein Gemisch aus 2,2'-Diphenylmethandiamin, 2,4'-Diphenylmethandiamin, 4,4'-Diphenylmethandiamin und aus mindestens einem Polyamin der Diphenylmethan-Reihe ist.

Gemäß einer bevorzugten Ausführungsform der vorliegenden Erfindung enthält das in (i) eingesetzte Amin ein Gemisch aus 2,2'-Diphenylmethandiamin, 2,4'-Diphenylmethandiamin, und 4,4'-Diphenylmethandiamin in einer Menge im Bereich von 1 bis 100, weiter bevorzugt von 20 bis 90 Gew.-%, weiter bevorzugt von 30 bis 80 Gew.%, weiter bevorzugt von 40 bis 75 Gew.%, und besonders bevorzugt von 55 bis 70 Gew.%, jeweils bezogen auf das Gesamtgewicht des Amins. Die jeweiligen Anteile der einzelnen Isomere können dabei variieren.

Gemäß einer Ausführungsform der Erfindung enthält das Amin 2,2'-Diphenylmethandiamin in einer Menge im Bereich von 0 bis 1 Gew.-% wie beispielsweise 0,0001 bis 1 Gew.%, 2,4'-Diphenylmethandiamin in einer Menge im Bereich von 4 bis 10 Gew.%, und 4,4'-Diphenylmethandiamin in einer Menge im Bereich von 40 bis 70 Gew.-%.

Enthält das Amin mindestens ein Polyamin der Diphenylmethan-Reihe, so enthält es dieses bevorzugt in einer Menge im Bereich von bis zu 100 Gew.- %, bevorzugt 1 bis 90 Gew.%, weiter bevorzugt in einer Menge im Bereich von 10 bis 80 Gew.%, und besonders bevorzugt in einer Menge im Bereich von 20 bis 55 Gew.%, jeweils bezogen das Gesamtgewicht des Amins. Enthält das Amin zwei oder mehr Polyamine, so beziehen sich die Gew.%-Angaben auf die Summe der Polyamine bezogen auf das Gesamtgewicht des Amins. Die jeweiligen Anteile der einzelnen Polyamine, können dabei variieren. Gemäß einer Ausführungsform der Erfindung enthält das Amin 3-Kem-MDA in einer Menge im Bereich von 15 bis 30 Gew.-% und hat einen Anteil an höheren Oligomeren in einer Menge im Bereich von 5 bis 25 Gew.-%.

Gemäß einer bevorzugten Ausführungsform enthält das Amin 2,2'-Diphenylmethandiamin in einer Menge im Bereich von 0 bis 1 Gew.-% wie beispielsweise 0,0001 bis 1 Gew.%, 2,4'-Diphenylmethandiamin in einer Menge im Bereich von 4 bis 10 Gew.%, und 4,4'-Diphenylmethandiamin in einer Menge im Bereich von 40 bis 70 Gew.%, Amin 3-Kern-MDA in einer Menge im Bereich von 15 bis 30 Gew.-%, und höhere Oligomere in einem Bereich von 5 bis 25 Gew.%, jeweils bezogen auf das Gesamtgewicht des Amins.

### Bereitstellung des Amins

Die Zusammensetzung des Amin ergibt sich typischerweise aus der Art der Bereitstellung. Im Allgemeinen kann das Amin auf jede dem Fachmann bekannte Art und Weise bereitgestellt werden.

Handelt es sich bei dem Amin entsprechend der oben beschriebenen bevorzugten Ausführungsform um mindestens ein Diamin und/oder mindestens ein Polyamin der Diphenylmethan-Reihe, so wird das Amin bevorzugt durch Umsetzen von Anilin mit Formaldehyd bereitgestellt.

Der Formaldehyd kann dem erfindungsgemäßen Verfahren in Form von wässrigem Formaldehyd oder in Form von höheren Homologen, so genannten Poly(oxy)methylen-Glykolen oder Paraformaldehyd oder Trioxan, aber auch anderen Formaldehydvorläufem wie z.B. Methylal, zugeführt werden. Bevorzugt wird der Formaldehyd als wässrige Lösung eingesetzt.

Wird der Formaldehyd als wässrige Lösung eingesetzt, so weist diese bevorzugt einen Wassergehalt im Bereich von 1 bis 95 Gew.%, weiter bevorzugt von 25 bis 90 Gew.-%, weiter bevorzugt von 40 Gew.-% bis 80 Gew.-% und besonders bevorzugt von 50 bis 70 Gew.-% auf, jeweils bezogen auf das Gesamtgewicht der wässrigen Lösung.

Bezüglich des bei der Umsetzung mit Formaldehyd eingesetzten Anilins bestehen im Allgemeinen keine Beschränkungen. Typischerweise wird das Anilin durch Nitrierung und Hydrierung aus Benzol großtechnisch hergestellt.

Bevorzugt wird bei der Umsetzung zur Bereitstellung des Amins ein molares Verhältnis von Anilin zu Formaldehyd im Bereich von 20:1 bis 1:1, weiter bevorzugt im Bereich von 10:1 bis 1,5:1, weiter bevorzugt im Bereich von 4:1 bis 1,75:1 und besonders bevorzugt im Bereich von 2,5:1 bis 1,8:1 eingesetzt.

Im Rahmen der vorliegenden Erfindung erfolgt diese Umsetzung bevorzugt in Gegenwart eines sauren Katalysators.

Demnach betrifft die vorliegende Erfindung auch ein Verfahren, wie oben beschrieben, wobei das Amin durch Umsetzen von Anilin und Formaldehyd in Gegenwart eines sauren Katalysators hergestellt wird. Weiterhin beschreibt die vorliegende Erfindung ein Isocyanat, herstellbar durch dieses Verfahren.

Bevorzugte Katalysatoren, die im Rahmen der Erfindung zur Umsetzung von Anilin mit Formaldehyd verwendet werden, sind homogene Säuren, insbesondere Mineralsäuren, wie beispielsweise Salzsäure, Schwefelsäure, Methansulfonsäure und Phosphorsäure und heterogene Säuren wie beispielsweise saure Zeolithe, Ionentauscher, Tone sowie Polysäuren. Ebenso können Gemische von zwei oder mehr der oben genannten Säuren verwendet werden. Die Verwendung von Salzsäure als Katalysator ist besonders bevorzugt. Wird als Katalysator Salzsäure verwendet, so kann die Säure auch in Form von gasförmigem Chlorwasserstoff eingesetzt werden. Die Katalysatormenge wird bevorzugt so gewählt, dass sich ein molares Verhältnis von Katalysator zu Anilin im Bereich von 0,01:1 bis 1:1, bevorzugt im Bereich von 0,05:1 bis 0,5:1 und besonders bevorzugt im Bereich von 0,08:1 bis 0,3:1 ergibt.

Weiterhin kann die Umsetzung in Gegenwart eines Lösungsmittels durchgeführt werden. Bevorzugt wird die Umsetzung in einem Lösungsmittel, ausgewählt aus der Gruppe bestehend aus Wasser, Dimethylformamid, Alkoholen, Ethern, Aromaten, aliphatischen Aminen, aromatischen Aminen und Gemischen aus zwei oder mehr dieser Verbindungen durchgeführt. Weiter bevorzugt wird die Umsetzung in einem Lösungsmittel, ausgewählt aus der Gruppe bestehend aus Wasser, Dimethylformamid, linearen C1-bis C18-Alkoholen, aliphatischen Aminen, aromatischen Aminen, Tetrahydrofuran, Diethylether, tert-Butylmethylether, Benzol, Toluol, Xylol, halogenierten Aromaten, wie beispielsweise Mono- oder Dichlorbenzol, und Gemischen aus zwei oder mehr dieser Verbindungen durchgeführt. Enthält das Lösungsmittel einen Alkohol, so enthält es bevorzugt einen linearen oder verzweigten C1- bis C18-Alkohol. Enthält das Lösungsmittel ein Amin, so enthält es bevorzugt ein aromatisches Amin, weiter bevorzugt das Amin, welches mit Formaldehyd umgesetzt wird. Besonders bevorzugt wird im Rahmen der Erfindung als Lösungsmittel Wasser und/oder ein aromatisches Amin, bevorzugt Anilin, als Lösungsmittel verwendet.

Gemäß einer besonders bevorzugten Ausführungsform wird die Umsetzung von Anilin mit Formaldehyd zur Bereitstellung des Amins im wässrigen Medium mit Salzsäure als Katalysator durchgeführt.

Gemäß einer Ausführungsform der Erfindung wird zur Bereitstellung des Amins Anilin, Formaldehyd, der eingesetzte Katalysator und gegebenenfalls das Lösungsmittel in einer geeigneten Mischvorrichtung, wie beispielsweise in einer Mischpumpe, einer Düse oder einem statischen Mischer, vermischt und in einer geeigneten Reaktionsvorrichtung, beispielsweise in einem Rohrreaktor, einem Rührreaktor oder einer Reaktionskolonne umgesetzt. Die Umsetzungstemperatur liegt bevorzugt im Bereich von 20 bis 200 °C, weiter bevorzugt im Bereich von 25 bis 170 °C, weiter bevorzugt im Bereich von 30 bis 150 °C, und besonders bevorzugt im Bereich von 40 und 140°C .

Gemäß einer alternativen Ausführungsform wird zunächst Anilin mit dem Katalysator und gegebenenfalls mit einem Lösungsmittel in einer geeigneten Mischvorrichtung vermischt; erst anschließend wird Formaldehyd bei einer Temperatur der Mischung bevorzugt im Bereich von 20 bis 120 °C, weiter bevorzugt im Bereich von 25 bis 100 °C, weiter, und besonders bevorzugt im Bereich von 30 und 90 °C, zugemischt und anschließend in einer geeigneten Reaktionsvorrichtung umgesetzt. Die Umsetzungstemperatur ist, wie oben beschrieben, bevorzugt im Bereich von 20 bis 200 °C, weiter bevorzugt im Bereich von 25 bis 170 °C, weiter bevorzugt im Bereich von 30 und 150 °C, und besonders bevorzugt im Bereich von 40 und 140°C.

Gemäß einer weiteren Ausführungsform wird zunächst Anilin mit Formaldehyd und gegebenenfalls mit einem Lösungsmittel in einer geeigneten Mischvorrichtung vermischt und bei einer Temperatur im Bereich von 5 bis 150 °C, bevorzugt im Bereich von 20 bis 120 °C und besonders bevorzugt im Bereich von 30 bis 90 °C umgesetzt. Erst anschließend wird das verbleibende Gemisch, gegebenenfalls nach geeigneten Zwischenschritten, wie beispielsweise Destillation oder Phasentrennung, mit dem Katalysator, bevorzugt bei einer Temperatur im Bereich von 20 bis 200 °C, in Kontakt gebracht. Die Umsetzung erfolgt wiederum bevorzugt bei einer Temperatur Bereich von 20 bis 200° C, weiter bevorzugt im Bereich von 25 bis 170 °C, weiter bevorzugt im Bereich von 30 und 150 °C, und besonders bevorzugt zwischen 40 und 140 °C.

Im Rahmen der vorliegenden Erfindung ist es möglich, die Temperatur im Verlauf der oben beschriebenen Umsetzungen zur Bereitstellung des Amins zu ändern. Hierbei ist es möglich, die Umsetzung bei zwei oder mehr Temperaturen bzw. in zwei oder mehr Temperaturbereichen durchzuführen, die jeweils in den oben angegeben Grenzen liegen. Temperaturänderungen im Laufe der Reaktion können kontinuierlich oder diskontinuierlich vollzogen werden. Bevorzugt wird die Temperatur während der Umsetzung erhöht, bevorzugt kontinuierlich erhöht.

Bevorzugt wird die erhaltene, das Amin enthaltende Reaktionsmischung im Anschluss an die Umsetzung in geeigneter Weise aufgereinigt.

Was die Aufreinigung der Reaktionsmischung betrifft, so bestehen generell keine Beschränkungen.

Gemäß einer bevorzugten Ausführungsform wird die jeweils erhaltene Reaktionsmischung zur Aufreinigung durch Zugabe mindestens einer geeigneten Base neutralisiert. Geeignete Basen sind beispielsweise Alkalimetallhydroxide wie Natriumhydroxid oder Kaliumhydroxid, Erdalkalimetallhydroxide oder Erdalkalioxide, oder Ammoniak. Bevorzugt wird die Reaktionsmischung mit Natriumhydroxid neutralisiert. Weiter bevorzugt erfolgt die Neutralisierung dabei in einem Temperaturbereich im Bereich von 20 bis 150 °C, weiter bevorzugt im Bereich von 30 bis 140 °C und besonders bevorzugt im Bereich von 40 bis 120 °C.

Wird die Reaktionsmischung durch Zugabe mindestens einer geeigneten Base neutralisiert, so wird bevorzugt in einem weiteren Schritt von dem so erhaltenen Gemisch die wässrige Phase unter Erhalt eines das Amin enthaltenden Gemischs abgetrennt. Wird eine Neutralisation mit sich anschließender Phasentrennung durchgeführt, so können diese einzelnen Schritte jeweils einmal oder mehrmals durchgeführt werden.

Gemäß einer alternativen Ausführungsform wird das Amin enthaltende Gemisch zur Aufreinigung, gegebenenfalls nach Durchführen der mindestens einen Neutralisation und sich anschließender Phasentrennung, destilliert. Die Destillation wird bevorzugt bei Temperaturen im Bereich von 100 bis 300 °C, bevorzugt im Bereich von 120 bis 260 °C durchgeführt. Bevorzugt erfolgt die Destillation bei einem Druck im Bereich von 1 bis 2000 mbar, weiter bevorzugt im Bereich von 2 bis 1500 mbar.

Die genaue Zusammensetzung des erhaltenen, gegebenenfalls aufgereingten, Amins ist abhängig von den Herstellungsbedingungen. So kann das Verhältnis der einzelnen Diamine und/oder Polyamine zueinander durch die Wahl unterschiedlicher Verfahrensbedingungen bei der Umsetzung von Anilin mit Formaldehyd in weiten Grenzen gesteuert werden. Die Polyamine der Diphenylmethan-Reihe bilden sich typischerweise dadurch, dass die Reaktion nicht beim Diamin, also beim Zweikernprodukt stehen bleibt, sondern, typischerweise in abnehmender Menge, zu den drei- und höherkemigen Produkten weiter reagiert.

Eine Steuerung im Hinblick auf den Erhalt einer bevorzugten Zusammensetzung erfolgt bevorzugt durch das Einstellen des jeweiligen Verhältnisses von Anilin zu Formaldehyd bzw. des jeweiligen Verhältnisses des sauren Katalysators zum Anilin.

Typischerweise wird bei der Bereitstellung des Amins durch Umsetzung von Formaldehyd mit Anilin das Amin im Gemisch mit weiteren Substanzen erhalten. Als weitere Substanzen sind beispielsweise nicht umgesetztes Anilin, nicht-umgesetzter Formaldehyd, Wasser sowie Nebenprodukte, wie beispielsweise Vorstufen zu farbgebenden Substanzen, zu nennen.

Das aus der Umsetzung von Anilin mit Formaldehyd erhaltene, gegebenenfalls wie oben beschrieben aufgereinigte und Amin enthaltende Gemisch kann direkt in (i) als Gemisch (Gi) eingesetzt werden. Der Begriff "direkt" bedeutet in diesem Zusammenhang, dass das aus der Umsetzung von Anilin mit Formaldehyd erhaltene Gemisch ohne weitere Zwischenbehandlung in (i) eingesetzt wird. Gemäß dieser Ausführungsform entspricht das aus der Umsetzung erhaltene, gegebenenfalls aufgereinigte und Amin enthaltende Gemisch dem Gemisch (Gi).

Gemäß einer alternativen Ausführungsform wird das aus der Bereitstellung erhaltene Gemisch vor (i) mindestens einer Zwischenbehandlung unter Erhalt des Gemischs (Gi) unterzogen.

Unter einer Zwischenbehandlung wird beispielsweise eine Destillation und/oder Kristallisation und/oder Neutralisation mit sich anschließender Wasserabtrennung des aus der Umsetzung erhaltenen, gegebenenfalls wie oben beschrieben aufgereinigten und Amin enthaltenden Gemisches verstanden.

Beispielsweise ist es möglich, als Zwischenbehandlung die Zusammensetzung des Amins durch Anreicherung einzelner Diamine und/oder Polyamine, oder Gemische aus einzelnen Diaminen, oder Gemische aus einzelnen Polyaminen, beispielsweise durch Destillation oder Kristallisation zu verändern. Weiterhin können einzelne Diamine und/oder Polyamine und/oder Gemische aus einzelnen Diaminen, oder Gemische aus einzelnen Polyaminen, abgetrennt oder zum Teil in reiner Form dargestellt werden, und die abgetrennten einzelnen Diamine und/oder Polyamine oder Gemische aus einzelnen Diaminen, oder Gemische aus einzelnen Polyaminen, in (i) eingesetzt werden.

### Gemisch (Gi)

Das Gemisch (Gi) enthält typischerweise mindestens eine Vorstufe zu einer farbgebenden Substanz. In diesem Zusammenhang bedeutet die Formulierung "Vorstufe zu einer farbgebenden Substanz", dass aus der Umsetzung dieser Substanz in Schritt (ii), also in der Phosgenierungsstufe, eine farbgebende Substanz resultiert. Beispielsweise wird die Vorstufe zu einer farbgebenden Substanz als Nebenprodukt bei der Umsetzung von Anilin mit Formaldehyd gebildet. Ohne auf konkrete Verbindungen beschränken zu wollen, seien beispielhaft für solche möglichen Vorstufen N-formylierte Diamine und/oder N-formylierte Polyamine der Diphenylmethan-Reihe oder Verbindungen der Stoffklasse der 3,4-Dihydrochinazoline zu nennen.

Unter einem N-formylierten Diamin bzw. Polyamin der Diphenylmethan-Reihe werden im Rahmen der Erfindung Diamine bzw. Polyamine der Diphenylmethan-Reihe verstanden, die an einer oder mehreren Aminfunktionalitäten formyliert sind, also Amine der folgenden Struktur 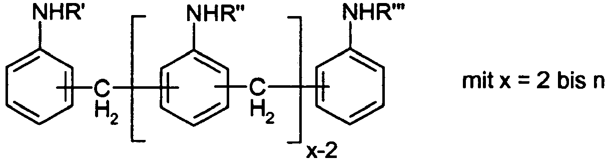 wobei R', R" und R"' unabhängig voneinander ausgewählt sind aus H und Formyl, wobei mindestens einer der Reste R' oder R" oder R"' Formyl ist und n wie oben definiert ist.

Beispielsweise kann das Gemisch (Gi) ein N-formyliertes Diamin ausgewählt aus den folgenden Strukturen enthalten: 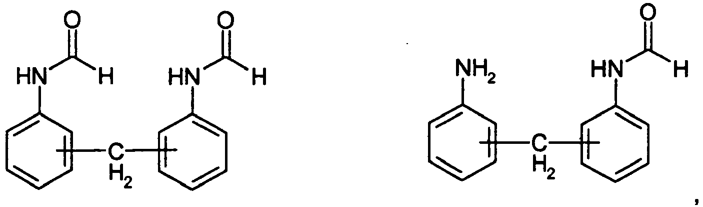 wie beispielsweise ein Diamin der folgenden Strukturen:

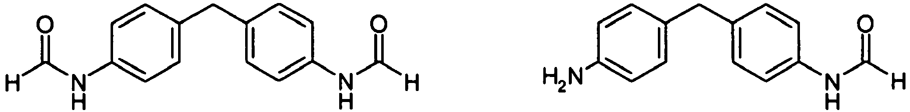

Unter einer Verbindung der Stoffklasse der 3,4-Dihydrochinazoline, die im Rahmen der Erfindung auch als 3,4-Dihydrazochinoline bezeichnet werden, werden Verbindungen der folgenden Struktur verstanden: 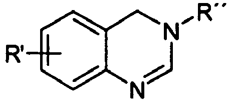 mit 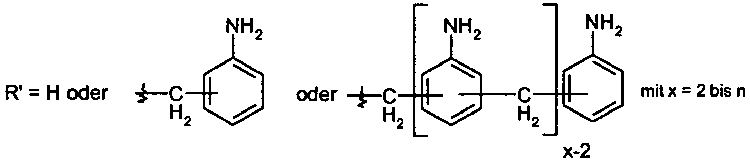 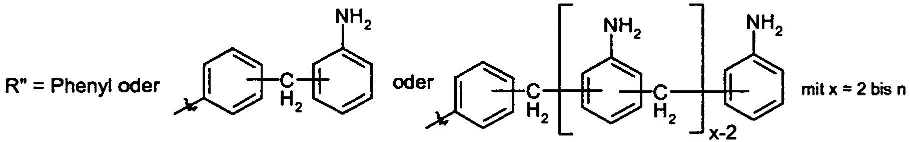 wobei x wie oben definiert ist. Beispielsweise enthält das Gemisch (Gi) eine oder mehrere Verbindungen, ausgewählt aus Verbindungen der folgenden Strukturen:

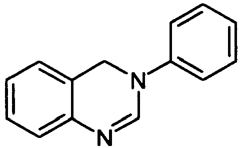

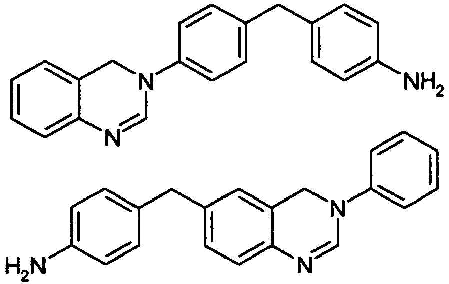

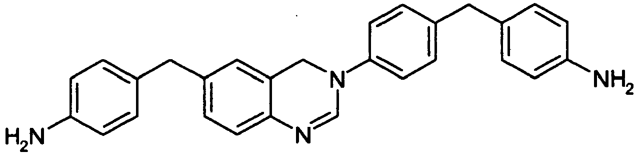

Demnach betrifft die vorliegende Erfindung auch ein wie oben beschriebenes Verfahren, wobei das Gemisch (Gi) zusätzlich zum Amin mindestens eine Verbindung enthält, ausgewählt aus der Gruppe bestehend aus N-formylierten Diaminen der Diphenylmethan-Reihe, N-formylierten Polyaminen der Diphenylmethan-Reihe und 3,4-Dihydrochinazolinen. Bevorzugt enthält das Gemisch (Gi) zusätzlich zum Amin mindestens ein N-formyliertes Diamin der Diphenylmethan-Reihe und mindestens eine Verbindung der Stoffklasse der 3,4-Dihydrochinazoline; weiter bevorzugt enthält das Gemisch (Gi) zusätzlich zum Amin mindestens ein N-formyliertes Diamin der Diphenylmethan-Reihe und mindestens eine Verbindung ausgewählt aus der Gruppe bestehend aus 3-Phenyl-3,4-Dihydrochinazolin, 3-(4-(4-Aminobenzyl)-phenyl)-3,4-Dihydrochinazolin, 6-(4-Aminobenzyl)-3-phenyl-3,4-Dihydrochinazolin und 6-(4-Aminobenzyl)-3-(4-(4-Aminobenzyl)-3,4-Dihydrochinazolin.

Gemäß einer bevorzugten Ausführungsform beschreibt die vorliegende Erfindung demnach auch ein Verfahren zur Herstellung eines Isocyanats, umfassend
(i) Hydrieren eines ein Amin und mindestens eine Vorstufe zu einer farbgebenden Substanz enthaltendes Gemisch (Gi), bevorzugt eines ein Amin und zusätzlich mindestens ein N-formyliertes Diamin der Diphenylmethan-Reihe und/oder N-formyliertes Polyamin der Diphenylmethan-Reihe und/oder mindestens eine Verbindung der Stoffklasse der 3,4-Dihydrochinazoline, enthaltenden Gemischs (Gi) in Anwesenheit eines Kupfer enthaltenden Hydrierkatalysators unter Erhalt eines das Amin enthaltenden Gemischs (Gii),
(ii) Umsetzen des Gemischs (Gii) mit Phosgen unter Erhalt eines das Isocyanat enthaltenden Gemischs (Giii).

Enthält das Gemisch (Gi) mindestens ein N-formyliertes Diamin der Diphenylmethan-Reihe, so enthält es dies bevorzugt in einer Menge von höchstens 5 Gew.%, bevorzugt in einer Menge von höchstens 2 Gew.%, und besonders bevorzugt in einer Menge von höchstens 1 Gew.%, jeweils bezogen auf das Gesamtgewicht des Gemisches (Gi) und, im Falle dass das Gemisch (Gi) mehr als ein N-formyliertes Diamin enthält, bezogen auf die Summe aller N-formylierten Diamine.

Enthält das Gemisch (Gi) mindestens ein N-formyliertes Polyamin der Diphenylmethan-Reihe, so enthält es dies bevorzugt in einer Menge von höchstens 3 Gew.%, bevorzugt in einer Menge von höchstens 2 Gew.-% und besonders bevorzugt in einer Menge von höchstens 1,5 Gew.%, jeweils bezogen auf das Gesamtgewicht des Gemisches (Gi)

Enthält das Gemisch ein oder mehrere Verbindungen der Stoffklasse der 3,4-Dihydrochinazoline, so enthält es diese in einer Menge von höchstens 2%, bevorzugt in einer Menge von höchsten 1 % und besonders bevorzugt in einer Menge von höchstens 0,5%, jeweils bezogen auf das Gesamtgewicht des Gemischs (Gi), wobei sich die Mengenangabe auf die Summe aller Verbindungen der Stoffklasse der 3,4-Dihydrochinazoline bezieht.

Demnach beschreibt die vorliegende Erfindung auch ein Verfahren, wobei das Gemisch (Gi) zusätzlich zum Amin Anilin und mindestens eine Verbindung ausgewählt aus der Gruppe bestehend aus N-formylierten Diaminen der Diphenylmethan-Reihe, N-formylierten Polyaminen der Diphenylmethan-Reihe und Verbindungen der Stoffklasse der 3,4-Dihydrochinazoline, enthält.

Weiterhin kann das Gemisch (Gi) zusätzlich zu dem Amin beispielsweise Anilin und/oder Wasser enthalten.

Weiterhin beschreibt die vorliegende Erfindung daher ein wie oben beschriebenes Verfahren, wobei das Gemisch (Gi) zusätzlich zum Amin Anilin oder Wasser oder sowohl Anilin als auch Wasser und mindestens eine Verbindung, ausgewählt aus der Gruppe bestehend aus N-formylierten Diaminen der Diphenylmethan-Reihe, N-formylierten Polyaminen der Diphenylmethan-Reihe und Verbindungen der Stoffklasse der 3,4-Dihydrochinazoline, enthält.

Enthält das Gemisch (Gi) Anilin, so enthält es dies üblicherweise in einer Menge im Bereich von bis zu 75 Gew.%, bevorzugt im Bereich von bis zu 50 Gew.%, besonders bevorzugt von bis zu 40 Gew.%, bezogen auf das Gesamtgewicht des Gemisches (Gi).

Enthält das Gemisch (Gi) Wasser, so enthält es dies üblicherweise in einer Menge im Bereich von bis zu 50 Gew.%, bevorzugt im Bereich von bis zu 25 Gew.%, besonders bevorzugt im Bereich von bis zu 10 Gew.%, bezogen auf das Gesamtgewicht des Gemisches (Gi).

Gemäß einer Ausführungsform enthält das Gemisch (Gi) Anilin in einer Menge im Bereich von bis zu 50 Gew.%, bevorzugt in einer Menge im Bereich von bis zu 40 Gew.-%, Wasser in einer Menge im Bereich von bis zu 25 Gew.%, bevorzugt von bis zu 10 Gew.%, mindestens ein N-formyliertes Diamin der Diphenylmethan-Reihe in einer Menge von höchstens 5 Gew.%, bevorzugt von höchstens 2 Gew.%, und besonders bevorzugt von höchstens 1 Gew.%, und mindestens eine Verbindungen der Stoffklasse der 3,4-Dihydrochinazoline in einer Menge von höchsten 2 Gew.%, bevorzugt von höchsten 1 Gew.-% und besonders bevorzugt von höchstens 0,5 Gew.%, jeweils bezogen auf das Gesamtgewicht des Gemischs (Gi).

Gemäß einer weiteren Ausführungsform enthält das Gemisch (Gi) kein Anilin oder kein Wasser oder weder Anilin noch Wasser.

### Schritt (i)

Die Hydrierung des Gemisches (Gi) gemäß (i) erfolgt bevorzugt bei einer Temperatur im Bereich von 20 bis 300 °C, besonders bevorzugt bei einer Temperatur von 40 bis 280 °C, weiter bevorzugt bei einer Temperatur im Bereich von 60 bis 240 °C und besonders bevorzugt bei einer Temperatur im Bereich von 70 bis 200 °C. Weiter bevorzugt sind Bereiche von 100 bis 220 °C, weiter bevorzugt von 120 bis 220 °C, und insbesondere bevorzugt von 120 bis 200 °C. Typischerweise erfolgt die Hydrierung gemäß (i) bei einem Druck im Bereich von 1 bis 300 bar, bevorzugt bei einem Druck im Bereich von 10 bis 100 bar und besonders bevorzugt bei einem Druck im Bereich von 20 bis 50 bar.

Demnach betrifft die vorliegende Erfindung auch ein Verfahren, wobei das Hydrieren gemäß (i) bei einer Temperatur im Bereich von 20 bis 300 °C, bevorzugt 70 bis 200 °C, weiter bevorzugt von 100 bis 220 °C, insbesondere bevorzugt von 120 bis 200 °C und bei einem Druck im Bereich von 1 bis 300 bar, bevorzugt 20 bis 50 bar erfolgt. Ebenso betrifft die vorliegende Erfindung ein Polyisocyanat, herstellbar nach diesem Verfahren.

Als Hydrierkatalysatoren in (i) kommen grundsätzlich alle geeigneten, Kupfer enthaltenden Hydrierkatalysatoren in Frage. Bevorzugt sind Hydrierkatalysatoren mit einem Kupfergehalt im Bereich von 0,1 bis 100 Gew.%, weiter bevorzugt im Bereich von 1 bis 80 Gew.%, weiter bevorzugt im Bereich von 2 bis 75 Gew.%, weiter bevorzugt im Bereich von 5 bis 70 Gew.%, weiter bevorzugt im Bereich von 10 bis 60 Gew.%, jeweils bezogen auf das Gesamtgewicht des Hydrierkatalysators und berechnet als Metall.

Demnach betrifft die vorliegende Erfindung auch ein Verfahren, wie oben beschrieben, wobei der Hydrierkatalysator Kupfer in einer Menge im Bereich von 0,1 bis 100 Gew.-%, bevorzugt im Bereich von 10 bis 60 Gew.%, bezogen auf das Gesamtgewicht des Hydrierkatalysators und berechnet als Metall, enthält. Ebenso betrifft die vorliegende Erfindung ein Polyisocyanat, herstellbar durch dieses Verfahren.

Das Kupfer kann dabei im Hydrierkatalysator als Metall oder als Kupfer-Verbindung oder als Gemisch aus mindestens zwei Kupfer-Verbindungen, oder als Gemisch aus Metall und einer Kupfer-Verbindung oder als Gemisch aus Metall und mindestens zwei Kupferverbindungen enthalten sein. Als Kupfer-Verbindungen sind beispielsweise bevorzugt Kupferchromat, Kupferoxid, Kupfernitrat, Kupfersulfat, Kupferhalogenide, wie beispielsweise Kupferchlorid, Kupferbromid oder Kupferiodid, Kupfercarbonat, Kupferacetylacetat, Kupferalkoxide oder Kupferaryloxide, sowie Kupfercarboxylate zu nennen.

Unter anderem bevorzugt werden im erfindungsgemäßen Verfahren geträgerte Hydrierkatalysatoren eingesetzt, die neben Kupfer mindestens ein Trägermaterial enthalten, wobei das Trägermaterial ein gegenüber den Reaktionsbedingungen inertes oder im wesentlichen inertes Trägermaterial sein kann. Das Trägermaterial ist bevorzugt ausgewählt aus der Gruppe bestehend aus Aktivkohle, Siliziumdioxid, Aluminiumoxide wie beispielsweise alpha-Aluminiumoxid, Zinkoxid, Titandioxid, Magnesiumoxid, Manganoxid, Zirkonoxid, Eisenoxid, Lanthanoxid und Mischungen aus zwei oder mehr dieser Materialien. Besonders bevorzugt ist das Trägermaterial ausgewählt aus der Gruppe bestehend aus Siliziumdioxid, Aluminiumoxid, Zinkoxid, Titandioxid, Manganoxid, Zirkonoxid, Lanthanoxid und einer Mischung aus zwei oder mehr dieser Materialien.

Der Begriff "Siliciumdioxid", wie er im Rahmen der vorliegenden Erfindung verwendet wird, umfasst auch Silikate wie beispielsweise Natriumsilikat.

Demnach betrifft die vorliegende Erfindung auch ein Verfahren, wie oben beschrieben, wobei der Hydrierkatalysator ein Trägermaterial, ausgewählt aus der Gruppe bestehend aus Siliziumdioxid, Aluminiumoxid, Zinkoxid, Titandioxid, Manganoxid, Zirkonoxid, Lanthanoxid und einer Mischung aus zwei oder mehr dieser Materialien, enthält.

Gemäß einer Ausführungsform des erfindungsgemäßen Verfahren enthält der Hydrierkatalysator das mindestens eine Trägermaterial in einer Menge von 1 bis 99 Gew.-%, bevorzugt von 10 bis 95 Gew.%, weiter bevorzugt von 25 bis 95 Gew.%, weiter bevorzugt von 40 bis 90 Gew.%, jeweils bezogen auf das Gesamtgewicht des Hydrierkatalysators.

Daher betrifft die vorliegende Erfindung auch das wie oben beschriebene Verfahren, wobei der Hydrierkatalysator das Trägermaterial in einer Menge im Bereich von 40 bis 90 Gew.%, bezogen auf das Gesamtgewicht des Hydrierkatalysators, enthält.

Gemäß einer bevorzugten Ausführungsform der vorliegende Erfindung enthält der Hydrierkatalysator beispielsweise Kupfer, bevorzugt in Form von Kupferoxid, in einer Menge im Bereich von 1 bis 80, Gew.%, bevorzugt im Bereich von 10 bis 70 Gew.%, und besonders bevorzugt im Bereich von 30 bis 65 Gew.%, jeweils bezogen auf das Gesamtgewicht des Hydrierkatalysators und berechnet als Metall, und Aluminiumoxid in einer Menge im Bereich von 20 bis 99 Gew.%, bevorzugt im Bereich von 30 bis 90 Gew.%, und besonders bevorzugt im Bereich von 35 bis 90 Gew.%, jeweils bezogen auf das Gesamtgewicht des Hydrierkatalysators.

Gemäß einer alternativen bevorzugten Ausführungsform der vorliegenden Erfindung enthält der Hydrierkatalysator beispielsweise Kupfer, bevorzugt in Form von Kupferoxid, in einer Menge im Bereich von 1 bis 80 Gew.%, bevorzugt im Bereich von 5 bis 60 Gew.%, weiter bevorzugt im Bereich von 10 bis 40 Gew.%, jeweils bezogen auf das Gesamtgewicht des Hydrierkatalysators und berechnet als Metall, und Siliciumdioxid in einer Menge im Bereich von 20 bis 99 Gew.%, bevorzugt im Bereich von 40 bis 95 Gew.%, und weiter bevorzugt im Bereich von 60 bis 90 Gew.%, jeweils bezogen auf das Gesamtgewicht des Hydrierkatalysators.

Im Rahmen der vorliegenden Erfindung kann der Hydrierkatalysator neben Kupfer und dem gegebenenfalls im Hydrierkatalysator enthaltenen mindestens einen Trägermaterial zusätzliche Elemente enthalten. Beispielsweise sind diese Elemente ausgewählt aus Elementen der Gruppen I bis VIII des Periodensystems der Elemente. Bevorzugt enthält der Hydrierkatalysator mindestens ein Element ausgewählt aus der Gruppe bestehend aus Lanthan, Magnesium, Mangan, Barium, Kohlenstoff, Chrom, Silber, Zink, Natrium, Gold und einer Mischung aus zwei oder mehr dieser Elemente.

Enthält der Hydrierkatalysator mindestens ein zusätzliches Element, so enthält er dieses bevorzugt in einer Menge im Bereich von 0,1 bis 50 Gew.%, bevorzugt im Bereich von 1 bis 40 Gew.%, jeweils bezogen auf das Gesamtgewicht des Hydrierkatalysators.

Gemäß einer Ausführungsform der vorliegenden Erfindung enthält der Hydrierkatalysator Kupfer, bevorzugt in Form von Kupferoxid, in einer Menge im Bereich von 1 bis 90 Gew.%, bevorzugt im Bereich von 10 bis 70 Gew.%, weiter bevorzugt im Bereich von 20 bis 50 Gew.%, jeweils bezogen auf das Gesamtgewicht des Hydrierkatalysators und berechnet als Metall , sowie Zink, bevorzugt in Form von Zinkoxid, in einer Menge im Bereich von 1 bis 90 Gew.%, bevorzugt im Bereich von 10 bis 70 Gew.%, weiter bevorzugt im Bereich von 20 bis 50 Gew.%, jeweils bezogen auf das Gesamtgewicht des Hydrierkatalysators und berechnet als Metall, und Aluminiumoxid in einer Menge im Bereich von 1 bis 60 Gew.%, bevorzugt im Bereich von 5 bis 50 Gew.%, weiter bevorzugt im Bereich von 10 bis 30 Gew.%, jeweils bezogen auf das Gesamtgewicht des Hydrierkatalysators.

Gemäß einer weiteren Ausführungsform der vorliegenden Erfindung enthält der Hydrierkatalysator Kupfer in einer Menge im Bereich von 10 bis 80 Gew.%, bevorzugt im Bereich von 20 bis 75 Gew.%, weiter bevorzugt im Bereich von 40 bis 70 Gew.-%, jeweils bezogen auf das Gesamtgewicht des Hydrierkatalysators und berechnet als Metall , sowie Lanthan, bevorzugt in Form von Lanthanoxid, in einer Menge im Bereich von 0,1 bis 20 Gew.%, bevorzugt im Bereich von 1 bis 10 Gew.-%, weiter bevorzugt im Bereich von 2 bis 6 Gew.%, jeweils bezogen auf das Gesamtgewicht des Hydrierkatalysators und berechnet als Metall, und Aluminiumoxid in einer Menge im Bereich von 1 bis 60 Gew.%, bevorzugt im Bereich von 5 bis 50 Gew.%, und besonders bevorzugt im Bereich von 10 bis 40 Gew.%, jeweils bezogen auf das Gesamtgewicht des Hydrierkatalysators. Besonders bevorzugt enthält der Hydrierkatalysator das Kupfer dabei als Gemisch aus Metall und Kupferoxid.

Gemäß einer weiteren Ausführungsform der vorliegenden Erfindung enthält der Hydrierkatalysator Kupfer, bevorzugt in Form von Kupferoxid, in einer Menge im Bereich von 1 bis 80 Gew.%, bevorzugt im Bereich von 10 bis 60 Gew.%, weiter bevorzugt im Bereich von 20 bis 45 Gew.%, jeweils bezogen auf das Gesamtgewicht des Hydrierkatalysators und berechnet als Metall, sowie Magnesium, bevorzugt in Form von Magnesiumoxid, in einer Menge im Bereich von 0,1 bis 50 Gew.%, bevorzugt im Bereich von 1 bis 25 Gew.%, weiter bevorzugt im Bereich von 5 bis 20 Gew.%, jeweils bezogen auf das Gesamtgewicht des Hydrierkatalysators und berechnet als Metall, und Siliciumdioxid in einer Menge im Bereich von 5 bis 80 Gew.%, bevorzugt im Bereich von 10 bis 60 Gew.%, weiter bevorzugt im Bereich von 20 bis 50 Gew.%, jeweils bezogen auf das Gesamtgewicht des Hydrierkatalysators.

Gemäß einer weiteren Ausführungsform der vorliegende Erfindung enthält der Hydrierkatalysator Kupfer, bevorzugt in Form von Kupferoxid und/oder metallischem Kupfer, in einer Menge im Bereich von 5 bis 90 Gew.%, bevorzugt im Bereich von 20 bis 80 Gew.%, weiter bevorzugt im Bereich von 40 bis 70 Gew.%, jeweils bezogen auf das Gesamtgewicht des Hydrierkatalysators und berechnet als Metall, sowie Mangan, bevorzugt in Form von Manganoxid, in einer Menge im Bereich von 0,1 bis 60 Gew.-%, bevorzugt im Bereich von 1 bis 40 Gew.%, weiter bevorzugt im Bereich von 5 bis 20 Gew.%, jeweils bezogen auf das Gesamtgewicht des Hydrierkatalysators und berechnet als Metall, und Aluminiumoxid in einer Menge im Bereich von 5 bis 80 Gew.-%, bevorzugt im Bereich von 10 bis 60 Gew.%, und besonders bevorzugt im Bereich von 20 bis 50 Gew.%, jeweils bezogen auf das Gesamtgewicht des Hydrierkatalysators. Besonders bevorzugt enthält der Hydrierkatalysator das Kupfer dabei als Gemisch aus metallischem Kupfer und Kupferoxid.

Besonders bevorzugt enthält der Hydrierkatalysator zusätzlich mindestens ein Element ausgewählt aus der Gruppe bestehend aus Barium, Chrom, Silber, Gold und Mischungen davon, weiter bevorzugt Barium, Chrom oder sowohl Barium als auch Chrom.

Enthält der Katalysator als mindestens ein zusätzliches Element Chrom, so liegt das molare Verhältnis von Kupfer zu Chrom bevorzugt im Bereich von 1:5 bis 100:1, weiter bevorzugt im Bereich von 1:3 bis 50:1, und weiter bevorzugt im Bereich von 1:2 bis 10:1, jeweils berechnet als Metall.

Gemäß einer Ausführungsform der vorliegende Erfindung enthält der Hydrierkatalysator Kupfer, bevorzugt in Form von Kupferoxid und Kupferchromat, in einer Menge im Bereich von 1 bis 80 Gew.%, bevorzugt im Bereich von 10 bis 70 Gew.%, weiter bevorzugt im Bereich von 25 bis 65 Gew.%, jeweils bezogen auf das Gesamtgewicht des Hydrierkatalysators und berechnet als metallisches Kupfer, sowie Chrom in einer Menge im Bereich von 1 bis 60 Gew.%, bevorzugt im Bereich von 5 bis 50 Gew.%, weiter bevorzugt im Bereich von 10 bis 40 Gew.%, jeweils bezogen auf das Gesamtgewicht des Hydrierkatalysators und berechnet als Metall, und Barium, bevorzugt in Form von Bariumoxid, in einer Menge im Bereich von 0,1 bis 40 Gew.%, bevorzugt im Bereich von 0,5 bis 30 Gew.%, weiter bevorzugt im Bereich von 1 bis 20 Gew.%, jeweils bezogen auf das Gesamtgewicht des Hydrierkatalysators und berechnet als Metall.

Gemäß einer weiteren Ausführungsform der vorliegende Erfindung enthält der Hydrierkatalysator Kupfer, bevorzugt in Form von Kupferoxid und Kupferchromat, in einer Menge im Bereich von 5 bis 70 Gew.%, bevorzugt im Bereich von 15 bis 60 Gew.-%, weiter bevorzugt im Bereich von 30 bis 50 Gew.%, jeweils bezogen auf das Gesamtgewicht des Hydrierkatalysators und berechnet als metallisches Kupfer, sowie Chrom in einer Menge im Bereich von 1 bis 60 Gew.%, bevorzugt im Bereich von 10 bis 50 Gew.%, weiter bevorzugt im Bereich von 20 bis 40 Gew.%, jeweils bezogen auf das Gesamtgewicht des Hydrierkatalysators und berechnet als Metall, und Kohlenstoff in einer Menge im Bereich von 0,1 bis 20 Gew.%, bevorzugt im Bereich von 0,5 bis 10 Gew.%, weiter bevorzugt im Bereich von 1 bis 5 Gew.%, jeweils bezogen auf das Gesamtgewicht des Hydrierkatalysators und berechnet als Element.

Gemäß einer weiteren Ausführungsform der vorliegende Erfindung enthält der Hydrierkatalysator Kupfer, bevorzugt in Form von Kupferoxid und Kupferchromat, in einer Menge im Bereich von 1 bis 80 Gew.%, bevorzugt im Bereich von 10 bis 60 Gew.-%, weiter bevorzugt im Bereich von 20 bis 40 Gew.%, jeweils bezogen auf das Gesamtgewicht des Hydrierkatalysators und berechnet als metallisches Kupfer, sowie Chrom in einer Menge im Bereich von 1 bis 70 Gew.%, bevorzugt im Bereich von 10 bis 50 Gew.%, weiter bevorzugt im Bereich von 20 bis 40 Gew.%, jeweils bezogen auf das Gesamtgewicht des Hydrierkatalysators und berechnet als Metall, und Kohlenstoff in einer Menge im Bereich von 0,1 bis 20 Gew.%, bevorzugt im Bereich von 0,5 bis 10 Gew.%, weiter bevorzugt im Bereich von 1 bis 5 Gew.%, jeweils bezogen auf das Gesamtgewicht des Hydrierkatalysators und berechnet als Element. Weiter bevorzugt enthält der Katalysator dieser Ausführungsform zusätzlich Barium und/oder Natrium, bevorzugt Barium und Natrium. Enthält der Katalysator zusätzlich Barium so enthält er dieses bevorzugt in einer Menge im Bereich von 0,1 bis 20 Gew.%, bevorzugt im Bereich von 1 bis 15 Gew.%, weiter bevorzugt im Bereich von 4 bis 10 Gew.%, jeweils bezogen auf das Gesamtgewicht des Hydrierkatalysators und berechnet als Metall. Enthält der Katalysator zusätzlich Natrium so enthält er dieses bevorzugt als Natriumsilikat in einer Menge im Bereich von 0,1 bis 20 Gew.%, bevorzugt im Bereich von 0,5 bis 15 Gew.%, jeweils bezogen auf das Gesamtgewicht des Hydrierkatalysators und berechnet als Natriumsilikat.

Gemäß einer weiteren Ausführungsform der vorliegende Erfindung enthält der Hydrierkatalysator Kupfer, bevorzugt in Form von Kupferoxid und Kupferchromat, in einer Menge im Bereich von 1 bis 80 Ges.-%, bevorzugt im Bereich von 5 bis 60 Gew.-%, weiter bevorzugt im Bereich von 20 bis 50 Gew.%, jeweils bezogen auf das Gesamtgewicht des Hydrierkatalysators und berechnet als metallisches Kupfer, sowie Chrom in einer Menge im von 1 bis 80 Gew.%, bevorzugt im Bereich von 5 bis 60 Gew.-%, weiter bevorzugt im Bereich von 20 bis 50 Gew.%, jeweils bezogen auf das Gesamtgewicht des Hydrierkatalysators und berechnet als Metall, und Kohlenstoff in einer Menge im Bereich von 0,1 bis 20 Gew.%, bevorzugt im Bereich von 0,5 bis 10 Gew.-%, weiter bevorzugt im Bereich von 1 bis 5 Gew.%, jeweils bezogen auf das Gesamtgewicht des Hydrierkatalysators und berechnet als Element. Weiter bevorzugt enthält der Katalysator dieser Ausführungsform zusätzlich Mangan und/oder Natrium, bevorzugt Mangan und Natrium. Enthält der Katalysator zusätzlich Mangan so enthält er dieses bevorzugt in einer Menge im Bereich von 0,1 bis 20 Gew.%, bevorzugt im Bereich von 0,5 bis 10 Gew.%, weiter bevorzugt im Bereich von 1 bis 5 Gew.%, jeweils bezogen auf das Gesamtgewicht des Hydrierkatalysators und berechnet als Metall. Enthält der Katalysator zusätzlich Natrium, so enthält er dieses bevorzugt in einer Menge im Bereich von 0,1 bis 10 Gew.%, bevorzugt im Bereich von 0,2 bis 5 Gew.-%, jeweils bezogen auf das Gesamtgewicht des Hydrierkatalysators und berechnet als Natriumsilikat.

Die erfindungsgemäß in (i) eingesetzten Hydrierkatalysatoren zeichnen sich durch besonders vorteilhafte Katalysatorstandzeiten aus.

Gemäß einer bevorzugten Ausführungsform wird der Hydrierkatalysator vor seiner Verwendung in (i) geeignet aktiviert. Die Aktivierung kann generell nach allen dem Fachmann bekannten und geeigneten Verfahren erfolgen. Bevorzugt wird der Katalysator durch Inkontaktbringen mit einem Gemisch, enthaltend Wasserstoff, aktiviert. Gegebenfalls enthält das Gemisch neben Wasserstoff mindestens ein Inertmedium, bevorzugt ein Inertmedium ausgewählt aus der Gruppe bestehend aus Stickstoff, Helium, Argon, und Mischungen aus zwei oder mehr dieser Verbindungen, weiter bevorzugt Stickstoff. Bevorzugt erfolgt dieses Inkontaktbringen bei einer Temperatur im Bereich von 100 bis 300°C, weiter bevorzugt bei einer Temperatur im Bereich von 125 bis 250°C, und besonders bevorzugt bei einer Temperatur im Bereich von 150 bis 200°C. Die Temperatur kann im Verlauf der Aktivierung in den genannten Bereichen kontinuierlich oder diskontinuierlich geändert werden.

Was die Zusammensetzung des zur Aktivierung des Katalysators verwendeten Gemischs betrifft, so wird der Anteil an Wasserstoff im Gemisch bevorzugt während des Aktivierens kontinuierlich oder diskontinuierlich wie beispielsweise schrittweise erhöht. Bevorzugt werden zur Aktivierung Gemische eingesetzt, enthaltend einen Volumenanteil von Wasserstoff im Inertmedium im Bereich von 0,1 bis 100%, bevorzugt im Bereich von 1 bis 100%.

Gemäß einer bevorzugten Ausführungsform wird der Hydrierkatalysator beispielsweise bei einer Temperatur im Bereich von 100 bis 300 °C, bevorzugt 125 bis 250 °C und besonders bevorzugt von 150 bis 200 °C zunächst mit einem Gemisch enthaltend Stickstoff und Wasserstoff mit einem Wasserstoffanteil im Bereich von 1 % bis 50%, bevorzugt im Bereich von 1 % bis 25%, anschließend mit einem Gemisch enthaltend Stickstoff und Wasserstoff mit einem Wasserstoffanteil im Bereich von 1% bis 99% bevorzugt im Bereich von 1 % bis 75%, und anschließend mit in wesentlichen reinen Wasserstoff in Kontakt gebracht.

Die Hydrierung kann nach üblichen, dem Fachmann bekannten Verfahren durchgeführt werden. So kann das Gemisch (Gi) vor dem Kontakt mit dem Hydrierkatalysator mit Wasserstoff versetzt werden, oder es kann Wasserstoff nach der Hydrierkatalysatorzugabe der Lösung zugegeben werden. Hierbei kann beispielsweise Wasserstoff nach der Hydrierkatalysatorzugabe auf das Gemisch (Gi) aufgepresst werden. Die Hydrierung kann kontinuierlich oder diskontinuierlich erfolgen, wobei eine kontinuierliche Fahrweise bevorzugt ist.

Wird das Hydrieren gemäß (i) diskontinuierlich durchgeführt, wird es üblicherweise für eine Zeit im Bereich von 1 bis 48 h, bevorzugt im Bereich von 4 bis 36 h, und besonders bevorzugt im Bereich von 6 bis 24 h durchgeführt. Dabei wird üblicherweise ein Massenverhältnis von Katalysator zu MDA im Bereich von 1000:1 bis 1:1, bevorzugt von 500:1 zu 1:1 gewählt.

Wird die Hydrierung kontinuierlich durchgeführt, erfolgt das Inkontaktbringen mit Wasserstoff bevorzugt mit einer GHSV im Bereich von 0,1 bis 1000 h⁻¹, bevorzugt im Bereich von 0,5 bis 500 h⁻¹, und besonders bevorzugt im Bereich von 1 bis 100 h⁻¹. Die GHSV (gas hourly space velocity) ist definiert als das pro Stunde einströmende Gasvolumen (in I), geteilt durch das Volumen des Katalysators (in I).

Weiterhin erfolgt das Inkontaktbringen des Katalysators mit dem Gemisch (Gi) bei einer LHSV im Bereich von 0,1 bis 20 kg/(l*h), bevorzugt im Bereich von 0,2 bis 15 kg/(l*h) und besonders bevorzugt im Bereich von 0,5 bis 10 kg/(l*h). Die LHSV (liquid hourly space velocity) ist definiert als die pro Stunde einströmende Masse des Gemisch (Gi) (in kg), geteilt durch das Volumen des Katalysators (in I).

Die Hydrierung gemäß (i) kann ohne Lösungsmittel oder in Gegenwart mindestens eines Lösungsmittels durchgeführt werden. Als Lösungsmittel können Alkohole, beispielsweise lineare oder verzweigte aliphatische oder aromatische, insbesondere lineare oder verzweigte aliphatische oder aromatische Alkohole mit 1 bis 18 C-Atomen, insbesondere mit 1 bis 10 C-Atomen, besonders bevorzugt mit 1 bis 5 C-Atomen, wie beispielsweise Methanol, Ethanol oder tert.-Butanol, sowie weiterhin Amine, Ether, Aromaten, halogenierte Aromaten, aromatische Amine, Dimethylformamid, Wasser, bevorzugt Benzol, Toluol, Ethylbenzol, Xylole, halogenierte Aromaten, Anilin, Wasser, und weiter bevorzugt Monochlorbenzol (MCB) oder Dichlorbenzole, wie 1,4-Dichlorbenzol, oder Mischungen aus zwei oder mehreren dieser Verbindungen verwendet werden. Wird in (i) ein Lösungsmittel verwendet so wird bevorzugt ein Lösungsmittel ausgewählt aus der Gruppe bestehend aus Methanol, Ethanol, tert.-Butanol, Benzol, Toluol, Ethylbenzol, ortho-Xylol, meta-Xylol, para-Xylol, Monochlorbenzol, 1,4-Dichlorbenzol, Anilin, Wasser, DMF und Mischungen aus zwei oder mehreren davon verwendet.

Besonders bevorzugt wird (i) ohne Zugabe eines Lösungsmittels durchgeführt.

Der Hydrierkatalysator kann als Festbett oder Suspensionskatalysator eingesetzt werden. Er kann dabei in jeder beliebigen Form ausgebildet sein, vorzugsweise in einer Form, die sich zum Einsatz in Festbett-, Fließbett- oder Wirbelbettreaktoren eignet. Die Hydrierung kann beispielsweise kontinuierlich in Sumpf- oder Rieselfahrweise über ein Katalysatorbett erfolgen.

Handelt es sich um einen Suspensionskatalysator, so kann die Durchmischung bei der Suspensionsfahrweise zum Beispiel mittels üblicher Rührer, Begasungsrührer oder auch durch statische Mischer erfolgen. Kontinuierlich kann die Hydrierung beispielsweise in Suspensionsfahrweise in einem Kreislaufsystem über Düseneinspeisung mit Ausschleusung des katalysatorfreien Produkts nach bekannten Verfahren erfolgen. Bei der kontinuierlichen Fahrweise über ein Katalysatorbett kann z. B. im Wasserstoff Gleich- oder Gegenstrom gearbeitet werden.

Wird der Hydrierkatalysator als Formkörper eingesetzt, so kann die Formgebung beispielsweise durch Tablettierung, Extrusion, Sprühtrocknung, Sprühgranulation oder Strangpressen erfolgen. Über die spezifische Geometrie der Formkörper entscheiden in der Regel die prozesstechnischen Anforderungen, die durch das Verfahren, in dem der Hydrierkatalysator eingesetzt werden soll, vorgegeben werden.

Bevorzugt wird die Hydrierung so lange durchgeführt, bis ein Gemisch (Gi) mit gewünschter Qualität erreicht wird. Der Begriff "gewünschte Qualität" bedeutet in diesem Zusammenhang, dass das Gemisch (Gii) nach Schritt (iii) eine bessere Farbe aufweist als das Gemisch (Gi) nach Schritt (iii).

Enthält das Gemisch (i) mindestens eine Verbindung der Stoffklasse der 3,4-Dihydrochinazoline, so wird durch die Hydrierung gemäß (i) bevorzugt mindestens ein Teil mindestens einer dieser Verbindungen mit Wasserstoff umgesetzt, so dass das gemäß (i) erhaltene Gemisch (Gii) eine geringere Menge an der mindestens einen Verbindung enthält als das Gemisch (Gi). Besonders bevorzugt enthält das gemäß (i) erhaltene Gemisch (Gii) keine nachweisbare Menge der mindestens einen Verbindung der Stoffklasse der 3,4-Dihydrochinazoline mehr. Der Begriff "nicht nachweisbar" bedeutet in diesem Zusammenhang, dass das Gemisch (Gii) höchstens 10 ppm, bevorzugt weniger als 10 ppm der jeweiligen Verbindungen enthält, wobei sich die Mengenangabe auf jede einzelne Verbindung bezieht, bestimmt über eine geeignete Gaschromtaographie (GC)-Methode.

Enthält das Gemisch (i) mindestens ein N-formyliertes Diamin der Diphenylmethan-Reihe und/oder N-formyliertes Polyamin der Diphenylmethan-Reihe, so wird durch die Hydrierung gemäß (i) bevorzugt ein Teil mindestens einer dieser Verbindungen mit Wasserstoff umgesetzt, so dass das gemäß (i) erhaltene Gemisch (Gii) bevorzugt eine geringere Menge an der mindestens einen Verbindung enthält als das Gemisch (Gi). Besonders bevorzugt enthält das gemäß (i) erhaltene Gemisch (Gii) keine nachweisbaren Mengen der formylierten Verbindungen. Der Begriff "nicht nachweisbar" bedeutet in diesem Zusammenhang, dass das Gemisch (Gii) höchstens 50 ppm, bevorzugt weniger als 50 ppm der jeweiligen Verbindungen enthält, wobei sich die Mengenangabe auf jede einzelne Verbindung bezieht, bestimmt über eine geeignete Gaschromtaographie (GC)-Methode.

Bevorzugt wird das mindestens eine N-formylierte Diamin bzw. das mindestens eine N-formylierte Polyamin durch Schritt (i) zu dem korrespondierenden Diamin bzw. Polyamin umgesetzt. Dies hat den Vorteil, dass diese Verbindungen dann nicht zu farbgebenden Substanzen, sondern zu dem erwünschten Isocyanat umgesetzt werden.

### Schritt (ii)

Gemäß einer Ausführungsform wird das gemäß (i) erhaltene Gemisch (Gii) direkt, d.h. ohne weitere Zwischenbehandlung in (ii) eingesetzt.

Gemäß einer alternativen Ausführungsform wird das Gemisch (Gii) vor dem Umsetzen mit Phosgen gemäß (ii) mindestens einer Zwischenbehandlung unterzogen.

Als Zwischenbehandlung sind beispielsweise Destillation und/oder Kristallisation zu nennen.

Enthält das Gemisch (Gii) beispielsweise unerwünschte Leichtsieder, beispielsweise Wasser oder Anilin, so kann beispielsweise mindestens ein Teil der Leichtsieder vor Schritt (ii) von (Gii) abgetrennt werden. Bevorzugt werden in (Gii) enthaltende Leichtsieder vor Schritt (ii) destillativ abgetrennt.

Wird die Hydrierung gemäß (i) in Anwesenheit mindestens eines Lösungsmittels durchgeführt, so wird beispielsweise bevorzugt mindestens ein Teil des Lösungsmittels, bevorzugt im Wesentlichen das gesamte Lösungsmittel, vom Gemisch (Gii) abgetrennt, bevor dieses in (ii) eingesetzt wird. Eine solche Abtrennung kann beispielsweise durch Destillation erfolgen. Ebenso ist es grundsätzlich möglich, das Lösungsmittel nicht abzutrennen und das gesamte Gemisch (Gii) in (ii) einzusetzen.

Weiterhin ist es möglich, das Gemisch (Gii) mit anderen MDA-Fraktionen abzumischen. So können z.B. ein oder mehrere Diamine oder auch Polyamine zugemischt werden, die beispielsweise in Form einer bereits hydrierten Fraktion bereitgestellt werden können oder beispielsweise durch Destillation oder Kristallisation aus (Gi) gewonnen werden können.

Bevorzugt wird das gemäß (i) erhaltene Gemisch (Gii) vor der Umsetzung mit Phosgen gemäß (ii) destillativ behandelt; weiter bevorzugt werden Leichtsieder und/oder Lösungsmittel durch mindestens eine Destillation abgetrennt.

Demnach beschreibt die vorliegende Erfindung auch ein Verfahren zur Herstellung eines Isocyanats, umfassend
(i) Hydrieren eines ein Amin enthaltenden Gemischs (Gi) in Anwesenheit eines Kupfer enthaltenden Hydrierkatalysators unter Erhalt eines das Amin enthaltenden Gemischs (Gii),
(ii) Umsetzen des Gemischs (Gii) mit Phosgen unter Erhalt eines das Isocyanat enthaltenden Gemischs (Giii),
wobei das gemäß (i) erhaltene Gemisch (Gii) vor der Umsetzung mit Phosgen gemäß (ii) mindestens einer Zwischenbehandlung, bevorzugt einer Destillation, unterzogen wird.

Die Umsetzung mit Phosgen erfolgt üblicherweise bei Temperaturen im Bereich von 20 bis 250 °C, bevorzugt im Bereich von 30 bis 200 °C, weiter bevorzugt im Bereich von 40 bis 175 °C und besonders bevorzugt im Bereich von 50 bis 150 °C.

Es ist möglich, die Temperatur im Verlauf des Umsetzens gemäß Schritt (ii) zu ändern. Hierbei ist es möglich, das Umsetzen bei zwei oder mehr Temperaturen bzw. in zwei oder mehr Temperaturbereichen durchzuführen, die jeweils in den oben angegeben Grenzen liegen. Temperaturänderungen im Laufe von (ii) können kontinuierlich oder diskontinuierlich vollzogen werden.

Die Umsetzung gemäß (ii) erfolgt bevorzugt bei Drücken im Bereich von 1 bar bis 50 bar, bevorzugt bei Drücken im Bereich von 1 bar bis 20 bar, weiter bevorzugt im Bereich von 1 bar bis 15 bar, besonders bevorzugt im Bereich zwischen 1 bar und 12 bar, insbesondere im Bereich von 1 bis 10 bar.

Es ist möglich, den Druck im Verlauf des Umsetzens gemäß Schritt (ii) zu ändern. Hierbei ist es möglich, die Umsetzung bei zwei oder mehr Drücken bzw. in zwei oder mehr Druckbereichen durchzuführen, die jeweils in den oben angegeben Grenzen liegen. Druckänderungen im Laufe der Reaktion können kontinuierlich oder diskontinuierlich vollzogen werden.

Bevorzugt erfolgt die Umsetzung von Phosgen gemäß (ii) in einem Reaktor, beispielsweise in einem Rührkessel, Rohrreaktor, oder Rohrbündelreaktor. Bevorzugt erfolgt die Umsetzung in einem Rohrreaktor.

Die Umsetzung mit Phosgen wird gegebenenfalls in Gegenwart mindestens eines Lösungsmittels durchgeführt. Als Lösungsmittel können alle für den Phosgenierungsprozess bekannten, inerten aromatischen, aliphatischen oder cyclischen Kohlenwasserstoffe bzw. halogenierte Kohlenwasserstoffe eingesetzt werden, in dem das jeweilige Isocyanat löslich ist und die unter den Reaktionsbedingungen der Phosgenierung nicht angegriffen werden. Beispiele solcher Lösungsmittel sind aromatische Verbindungen wie z. B. Mono- und Dichlorbenzol, Toluole, Xylole und Naphthalinderivate, Alkane mit 5 bis 12 Kohlenstoffatomen wie z. B. Hexan, Heptan, Octan, Nonan, Decan, Cycloalkane wie z. B. Cyclohexan, inerte Ester und inerte Ether wie z. B. Ethyl- oder Butylacetat, Tetrahydrofuran, Dioxan oder Diphenylether. Besonders bevorzugt wird ein Lösungsmittel eingesetzt, ausgewählt aus der Gruppe bestehend aus Chlorbenzole und Kohlenwasserstoffen. Besonders bevorzugt wird Monochlorbenzol als Lösungsmittel eingesetzt.

Demnach betrifft die vorliegende Erfindung auch ein Verfahren, wie oben beschrieben, wobei (ii) in Gegenwart eines Lösungsmittels, bevorzugt in Gegenwart eines Lösungsmittels ausgewählt aus der Gruppe bestehend aus Chlorbenzolen und Kohlenwasserstoffen, ganz besonders bevorzugt in Monochlorbenzol durchgeführt wird. Ebenso betrifft die vorliegende Erfindung ein Isocyanat, herstellbar nach diesem Verfahren.

Im erfindungsgemäßen Verfahren wird in (ii) bevorzugt ein molares Verhältnis von Phosgen zu Amin im Bereich von 1:1 bis 15:1, bevorzugt 1,2:1 bis 10:1, besonders bevorzugt 1,3:1 bis 6:1, gewählt.

Die Umsetzung gemäß (ii) kann gegebenenfalls in Gegenwart mindestens eines inerten Mediums durchgeführt werden. Bei dem Inertmedium handelt es sich um ein Medium, das bei der Reaktionstemperatur üblicherweise gasförmig vorliegt und nicht mit den Edukten reagiert. Das Inertmedium wird im Allgemeinen vor der Umsetzung mit dem Gemisch (Gii) und/oder Phosgen vermischt. Bevorzugt ist das Inertmedium ausgewählt aus der Gruppe bestehend aus Stickstoff, Helium, Argon, SF6 und Mischungen aus zwei oder mehr dieser Verbindungen. Wird ein Inertmedium verwendet, wird besonders bevorzugt Stickstoff eingesetzt.

Wird ein Inertmedium verwendet, so wird dieses bevorzugt in einer Menge im Bereich von 0,1 bis 99,9 Volumenprozent, bevorzugt im Bereich von 1,0 bis 99,0 Volumenprozent, und besonders bevorzugt im Bereich von 10 bis 90 Volumenprozent, jeweils bezogen auf das Gesamtvolumen des mit Phosgen umzusetzenden Gemisches (Gii) eingesetzt.

Demnach beschreibt die vorliegende Erfindung auch ein Verfahren zur Herstellung eines Isocyanats, umfassend
(i) Hydrieren eines ein Amin enthaltenden Gemischs (Gi) in Anwesenheit eines Kupfer enthaltenden Hydrierkatalysators unter Erhalt eines das Amin enthaltenden Gemischs (Gii),
(ii) Umsetzen des Gemischs (Gii) mit Phosgen in Gegenwart mindestens eines inerten Mediums unter Erhalt eines das Isocyanat enthaltenden Gemischs (Giii).

Das Umsetzen gemäß (ii) kann kontinuierlich oder diskontinuierlich durchgeführt werden.

Wird das Umsetzen gemäß (ii) diskontinuierlich durchgeführt, wird es üblicherweise für eine Zeit im Bereich von 30 Sekunden bis 10 Stunden, bevorzugt im Bereich von 1 min bis 5 Stunden, und besonders bevorzugt im Bereich von 2 min bis 2 Stunden durchgeführt.

Wird das Umsetzen gemäß (ii) kontinuierlich durchgeführt, erfolgt das Umsetzen mit Phosgen bevorzugt mit einer GHSV im Bereich von 3,6 * 10⁵ h⁻¹ bis 10 h⁻¹, bevorzugt im Bereich von 3,6 * 10⁶ h⁻¹ bis 1 h⁻¹, weiter bevorzugt im Bereich von 1,08 * 10⁷ h⁻¹ bis 1.800 h⁻¹. Ebenfalls bevorzugt erfolgt das Umsetzen mit Phosgen mit einer GHSV (Gas Hourly Space Velocity) im Bereich von 1,08 * 10⁷ h⁻¹ bis 1 h⁻¹, bevorzugt im Bereich von 3,6 * 10⁶ h⁻¹ bis 10 h⁻¹, weiter bevorzugt im Bereich von 3,6 * 10⁵ h⁻¹ bis 1.800 h⁻¹.

Der im Rahmen der vorliegenden Erfindung verwendete Begriff "Isocyanat" bezeichnet, entsprechend dem oben erläuterten Begriff "Amin", mindestens ein Polyisocyanat und/oder mindestens ein Diisocyanat. Demnach bezeichnet der Begriff "Isocyanat" beispielsweise ein Diisocyanat, oder ein Polyisocyanat, oder ein Gemisch aus einem Diisocyanat und einem Polyisocyanat, oder ein Gemisch aus mindestens zwei Diisocyanaten, oder ein Gemisch aus mindestens zwei Polyisocyanaten, oder Gemisch mindestens zwei Diisocyanaten und aus mindestens zwei Polyisocyanaten, oder ein Gemisch aus mindestens zwei Diisocyanaten und aus einem Polyisocyanat, oder ein Gemisch aus mindestens zwei Diisocyanaten und mindestens zwei Polyisocyanaten.

Besonders bevorzugt ist das Isocyanat ausgewählt aus einem Diisocyanat der Diphenylmethan-Reihe, aus einem Gemisch aus zwei oder mehreren Diisocyanaten der Diphenylmethan-Reihe, aus einem Polyisocyanat der Diphenylmethan-Reihe, aus einem Gemisch aus mindestens zwei Polyisocyanaten der Diphenylmethan-Reihe, und aus einem Gemisch aus ein oder mehreren Diphenyldiisocyanaten der Diphenylmethan-Reihe mit ein oder mehreren Polyisocyanaten der Diphenylmethan-Reihe.

Ganz besonders bevorzugt ist das Isocyanat ein Gemisch aus mindestens zwei Diisocyanaten der Diphenylmethan-Reihe und mindestens zwei Polyisocyanaten der Diphenylmethan-Reihe.

Demnach beschreibt die vorliegende Erfindung auch ein Verfahren, wie oben beschrieben, wobei das Amin ein Gemisch aus zwei oder mehr Diaminen der Diphenylmethan-Reihe und zwei oder mehr Polyaminen der Diphenylmethan-Reihe ist und das Isocyanat ein Gemisch aus zwei oder mehr Diisocyanaten der Diphenylmethan-Reihe und zwei oder mehr Polyisocyanaten der Diphenylmethan-Reihe ist.

Ebenso betrifft die vorliegende Erfindung ein Isocyanat, herstellbar nach dem Verfahren, wobei das Isocyanat ein Gemisch aus zwei oder mehr Diisocyanaten der Diphenylmethan-Reihe und zwei oder mehr Polyisocyanaten der Diphenylmethan-Reihe ist.

Bei der Umsetzung gemäß (ii) kann das Isocyanat im Gemisch mit weiteren Bestandteilen entstehen.

Das gemäß (ii) erhaltene Gemisch enthält dabei üblicherweise weitere Bestandteile, wie z. B Lösungsmittel und/oder Phosgen und/oder Chlorwasserstoff und/oder Verunreinigungen, wie z.B. geringe Mengen an farbgebenden Substanzen oder Chlor bzw. chlorhaltigen Verbindungen. Als Beispiel für chlorhaltige Verbindungen sind etwa Carbamoylchloride zu nennen.

Das erhaltene Isocyanat-Gemisch kann auf übliche, dem Fachmann bekannte Art und Weise aufgearbeitet werden. Beispielsweise kann das Gemisch mit einem Lösungsmittel gewaschen werden. Als bevorzugte Lösungsmittel sind etwa Kohlenwasserstoffe, wie beispielsweise Chlorbenzol, Dichlorbenzol oder Toluol zu nennen.

Weiterhin können die weiteren Bestandteile oder zumindest ein Teil der weiteren Bestandteile in geeigneter Weise vom Polyisocyanat abgetrennt werden. Beispielsweise können die weiteren Bestandteile mittels mindestens einer Destillation vom Polyisocyanat abgetrennt werden. Der Begriff "mindestens eine Destillation" bedeutet in diesem Zusammenhang, dass eine oder mehre Destillationen durchgeführt werden können, die jeweils bei gleichen oder verschiedenen Temperaturen sowie in gleichen oder unterschiedlichen Druckbereichen durchgeführt werden können. Wird in (ii) ein Lösungsmittel eingesetzt, so wird bevorzugt zumindest das Lösungsmittel vom gemäß (ii) erhaltenen Gemisch durch Destillation abgetrennt.

Das gemäß (ii) erhaltene, gegebenenfalls in geeigneter Weise aufgereinigte Isocyanat hat bevorzugt einen Totalchlorgehalt von höchstens 9000 ppm, weiter bevorzugt von höchstens 8000 ppm, weiter bevorzugt von höchstens 7000 ppm, und insbesondere bevorzugt von höchstens 6000 ppm.

Demnach betrifft die vorliegende Erfindung auch ein Polyisocyanat, herstellbar nach einem Verfahren, wie oben beschrieben, mit einem Totalchlorgehalt, bestimmt gemäß ASTM D4661-98, von höchstens 9000 ppm, besonders bevorzugt von höchstens 6000 ppm.

Weiterhin hat das gemäß (ii) erhaltene, gegebenenfalls in geeigneter Weise aufgereinigte Isocyanat bevorzugt einen Gehalt an schwer hydrolysierbarem Chlor (DHC - "difficult hydrolysable chlorine") von höchstens 9000 ppm, bevorzugt von höchstens 5000, weiter bevorzugt von höchstens 4000 ppm, bestimmt gemäß ASTM D 4663-87.

Weiterhin hat das gemäß (ii) erhaltene, gegebenenfalls in geeigneter Weise aufgereinigte Isocyanat bevorzugt einen Gehalt an leicht hydrolysierbarem Chlor (EHC - "easily hydrolysable chlorine") von höchstens 1000 ppm, bevorzugt von höchstens 500 ppm, weiter bevorzugt von höchstens 300 ppm, bestimmt gemäß ASTM D 4667-87.

Demnach beschreibt die vorliegende Erfindung auch ein Isocyanat, herstellbar nach dem Verfahren, wie oben beschrieben, mit einem Totalchlorgehalt, bestimmt gemäß ASTM D4661-98, von höchstens 6000 ppm, mit einem Gehalt an schwer hydrolysierbarem Chlor (DHC - "difficult hydrolysable chlorine"), bestimmt gemäß ASTM D 4663-87, von höchstens 4000 ppm, und mit einem Gehalt an leicht hydrolysierbarem Chlor (EHC - "easily hydrolysable chlorine"), bestimmt gemäß ASTM D 4667-87, von höchstens 300 ppm.

Die erfindungsgemäßen Polyisocyanate haben bevorzugt eine Farbzahl L*, gemessen nach DIN 5033, von mindestens 90, bevorzugt von mindestens 92,5, besonders bevorzugt von mindestens 95.

Demnach betrifft die vorliegende Erfindung auch ein Polyisocyanat, herstellbar nach einem Verfahren, wie oben beschrieben, mit einem L*-Wert von mindestens 95, bestimmt gemäß DIN 5033.

Bevorzugt haben die erfindungsgemäßen Polyisocyanate eine Farbzahl b*, gemessen nach DIN 5033, von höchstens 40, bevorzugt von höchstens 30, ganz besonders bevorzugt von höchstens 25.

Demnach betrifft die vorliegende Erfindung auch ein Polyisocyanat, herstellbar nach einem Verfahren, wie oben beschrieben, mit einem b*-Wert von höchstens 25, bestimmt gemäß DIN 5033.

Demnach betrifft die vorliegende Erfindung auch ein Isocyanat, herstellbar nach einem Verfahren wie oben beschrieben, mit einer Farbzahl, gemessen gemäß DIN 5033, mit einem b*-Wert von höchstens 25 und einem L*-Wert von mindestens 95.

Besonders bevorzugt werden im Rahmen der vorliegenden Erfindung gleichzeitig bevorzugte Werte für den Totalchlorgehalt, EHC, DHC, b* und L* dann erhalten, wenn die Hydrierung in Schritt (i) bei Temperaturen im Bereich von 100 bis 220 °C, weiter bevorzugt im Bereich von 120 bis 220 °C und insbesondere 120 bis 200 °C gewählt werden. Beispielhaft sind etwa folgende bevorzugte Temperaturbereiche zu nennen: 100 bsi 220 °C oder 120 bis 220 °C oder 140 bis 220 °C oder 160 bis 220 °C oder 180 bis 220 °C oder etwa 100 bis 200 °C oder 120 bis 200 °C oder 140 bis 200 °C oder 160 bis 200 °C oder 180 bis 200 °C.

Grundsätzlich können die erfindungsgemäßen Polyisocyanate oder die erfindungsgemäß hergestellten oder herstellbaren Polyisocyanate zu sämtlichen denkbaren Verbindungen umgesetzt werden. Besonders bevorzugt werden die erfindungsgemäßen Polyisocyanate oder die erfindungsgemäß hergestellten oder herstellbaren Polyisocyanate als Ausgangsmaterialen zur Herstellung von Polyurethanen eingesetzt.

Daher beschreibt die vorliegende Erfindung auch die Verwendung der erfindungsgemäßen Polyisocyanate und/oder der erfindungsgemäß hergestellten oder herstellbaren Polyisocyanate zur Herstellung von Polyurethanen.

Die Erfindung soll anhand der nachstehenden Beispiele näher erläutert werden.

### Beispiele:

### I. Allgemeine Vorschrift

### I.1 Hydrierung

In einem Rohrreaktor wurden 50 ml eines Katalysators eingebaut und aktiviert. Der jeweilige Katalysator ist bezüglich seiner Zusammensetzung in den untenstehenden Beispielen und Vergleichsbeispielen explizit angegeben.

Alle Kupfer- und Nickel/Kupfer-Katalysatoren wurden zur Aktivierung bei jeweils 180°C für 18 h mit 1,5 Nl/h (Normliter/h) H₂ und 100 Nl/h N₂, anschließend für 3 Stunden mit 10 Nl/h H₂ und 100 Nl/h N₂ und abschließend für 3 Stunden mit 10 Nl/h H₂ behandelt.

Alle Palladium-, Ruthenium- und Rhodium-Katalysatoren wurden zur Aktivierung jeweils bei Raumtemperatur für 18 h mit 2 Nl/h H₂ und 50 Nl/h N₂, anschließend für 3 Stunden mit 10 Nl/h H₂ und 50 Nl/h N₂ und abschließend für 3 Stunden mit 10 Nl/h H₂ behandelt.

Anschließend an die Aktivierung wurden kontinuierlich 25 ml/h MDA in Sumpffahrweise über den Katalysator bei 30 bar Druck und bei verschiedenen Temperaturen geleitet. Wasserstoff wurde mit 2 Nl/h zudosiert. Im Falle, dass MDA eingesetzt wurde, das Anilin und/oder Wasser enthielt, wurde der Austrag an einem Rotationsverdampfer (1 mbar, 160°C, 2h) von Wasser und Anilin befreit und anschließend nach untenstehender Vorschrift I.2 phosgeniert. Im Falle, dass MDA ohne Leichtsieder eingesetzt wurde, wurde es direkt nach Vorschrift 1.2 phosgeniert.

### I.2 Phosgenierung

In ein Vorlagegefäß wurden 140 g gasförmiges Phosgen in 1300 ml MCB einkondensiert und anschließend auf 35 bis 50 °C erwärmt.

Aus einem Vorratsbehälter wurden unter Argon 70,0 g MDA in 1300 ml MCB in das Vodagegefäß eingebracht. Nach der Zugabe des Amins wurde die Temperatur auf 105 bis 125 °C erhöht. Nachdem sich alle Feststoffe gelöst hatten, wurde Phosgen bei Normaldruck abdestilliert. Anschließend wurde das MCB bei einem Druck von 100 mbar und bei 60 °C abdestilliert.

Nach dem Belüften mit Argon wurde das Roh-Isocyanat bei einer Ölbadtemperatur von 100 °C und 10 mbar Druck von restlichem MCB befreit und danach bei 10 mbar für 45 min auf 100 °C erwärmt. Danach wurde bei 10 mbar für 1 h auf 180 °C erwärmt.

Von der so erhaltenen Probe wurde die Farbzahl gemäß DIN 5033 mit einem Dr. Lange LICO 200 Messgerät gemessen und der Chlorgehalt bestimmt, wobei der Totalchlorgehalt (TC) gemäß ASTM D4661-98, der Gehalt an schwer zu hydrolysierendem Chlor (DHC - "difficult hydrolysable chlorine") gemäß ASTM D 4663-87, und der Gehalt an leicht zu hydrolysierendem Chlor (EHC - "easily hydrolysable chlorine") gemäß ASTM D 4667-87 bestimmt wurde.

### Vergleichsbeispiel 1:

Unbehandeltes MDA (MDA-1; 0,17 Gew.-% 2,2'-MDA; 4,25 Gew.-% 2,4'-MDA; 41,13 Gew.-% 4,4'-MDA; 19,25 Gew.-% 3-Kern-MDA; 18,78 Gew.-% höhere Oligomere; 13,9 Gew.-% Anilin; 2,2 Gew.-% Wasser; 0,12 Gew.-% N-Formyl-MDA; 449 ppm 3,4-Dihydrochinazoline) wurde nach der Standardvorschrift I.2 phosgeniert. Es wurde MDI mit Farbzahlen L*=74,0 und b*=42,8 und folgenden Chlorgehalten erhalten: TC 2000 ppm, DHC 800 ppm, EHC 150 ppm.

### Vergleichsbeispiel 2:

Unbehandeltes Roh-MDA (MDA-2; 0,22 Gew.-% 2,2'-MDA; 5,26 Gew.-% 2,4'-MDA; 50,44 Gew.-% 4,4'-MDA; 23,68 Gew.-% 3-Kern-MDA; 23,31 Gew.-% höhere Oligomere; 0,16 Gew.-% N-Formyl-MDA; 421 ppm 3,4-Dihydrochinazoline) wurde wie in Vergleichsbeispiel 1 behandelt. Es resultierten Farbzahlen von L*=74,2 und b*=58,1. Es wurden folgende Chlorgehalte erhalten: TC 1700 ppm, DHC 800 ppm, EHC 220 ppm.

### Vergleichsbeispiel 3:

Unbehandeltes Roh-MDA (MDA-3; 0,50 Gew.-% 2,2'-MDA; 9,13 Gew.-% 2,4'-MDA; 58,23 Gew.-% 4,4'-MDA; 21,00 Gew.-% 3-Kern-MDA; 10,20 Gew.-% höhere Oligomere; 0,12 Gew.-% N-Formyl-MDA; 512 ppm 3,4-Dihydrochinazoline) wurde wie in Vergleichsbeispiel 1 behandelt. Es resultierten Farbzahlen von L*=71,0 und b*=60,2. Es wurden folgende Chlorgehalte erhalten: TC 1800 ppm, DHC 900 ppm, EHC 190 ppm.

Demnach resultiert die Umsetzung des Amins in Schritt (ii) ohne Vorbehandlung zwar in akzeptablen Chlorwerten, aber in schlechter Farbe.

### Vergleichsbeispiel 4:

Die Herstellung erfolgte nach der allgemeinen Vorschrift I.1 und I.2, wobei als Katalysator in I.1 0,25 Gew.-% Palladium auf Aluminiumoxid eingesetzt wurde. Als MDA wurde MDA-1 eingesetzt. Die bei den jeweiligen Temperaturen erhaltenen Farbzahlen und Chlorwerte sind in der folgenden Tabelle angegeben:

| Hydriertemperatur [°C] | L* | b* | TC [ppm] | DHC [ppm] | EHC [ppm] |
|---|---|---|---|---|---|
| 140 | 89,9 | 53,2 | 8100 | 1800 | 270 |
| 160 | 92,4 | 41,8 | 12100 | 4600 | 510 |
| 180 | 92,8 | 53,9 | 16400 | 7500 | 960 |
| 200 | 89,2 | 80,0 | 22300 | 14300 | 1800 |
| 220 | 87,2 | 93,1 | 22400 | 14500 | 1900 |

### Vergleichsbeispiel 5:

Die Herstellung erfolgte nach der allgemeinen Vorschrift I.1 und 1.2, wobei als Katalysator in 1.1 0,15% Pd auf Aluminiumoxid eingesetzt wurde. Als MDA wurde MDA-1 eingesetzt. Die bei den jeweiligen Temperaturen erhaltenen Farbzahlen und Chlorwerte sind in der folgenden Tabelle angegeben:

| Hydriertemperatur [°C] | L* | b* | Totalchlor [ppm] | DHC [ppm] | EHC [ppm] |
|---|---|---|---|---|---|
| 100 | 92,1 | 69,4 | 6800 | 1200 | 250 |
| 120 | 92,5 | 54,8 | 8400 | 3800 | 310 |
| 140 | 92,8 | 44,4 | 9000 | 4100 | 320 |
| 160 | 92,2 | 48,6 | 12000 | 6900 | 840 |
| 180 | 92,2 | 68,0 | 16500 | 9000 | 1220 |

### Vergleichsbeispiel 6:

Die Herstellung erfolgte nach der allgemeinen Vorschrift I.1 und 1.2, wobei als Katalysator in I.1 5% Ruthenium auf Titandioxid eingesetzt wurde. Als MDA wurde MDA-1 eingesetzt. Die bei den jeweiligen Temperaturen erhaltenen Farbzahlen und Chlorwerte sind in der folgenden Tabelle angegeben:

| Hydriertemperatur [°C] | L* | b* | Totalchlor [ppm] | DHC [ppm] | EHC [ppm] |
|---|---|---|---|---|---|
| 95 | 84,3 | 48,2 | 4500 | 900 | 200 |
| 110 | 89,8 | 47,0 | 6400 | 1100 | 250 |
| 125 | 84,8 | 47,8 | 7100 | 1200 | 270 |

Außerdem wurden bis zu 3% kernhydrierte Produkte detektiert.

### Vergleichsbeispiel 7:

Die Herstellung erfolgte nach der allgemeinen Vorschrift I.1 und I.2, wobei als Katalysator in I.1 1% Rhodium auf Kohle eingesetzt wurde. Als MDA wurde MDA-2 eingsetzt. Die bei den jeweiligen Temperaturen erhaltenen Farbzahlen und Chlorwerte sind in der Tabelle angegeben.

| Hydriertemperatur [°C] | L* | b* | Totalchlor [ppm] | DHC [ppm] | EHC [ppm] |
|---|---|---|---|---|---|
| 100 | 77,2 | 53,6 | 1700 | 880 | 220 |
| 120 | 79,1 | 70,8 | 2000 | 940 | 230 |
| 140 | 80,9 | 46,9 | 2100 | 1380 | 200 |
| 160 | 85,4 | 46,1 | 2100 | 1560 | 250 |
| 180 | 86,2 | 40,9 | 2200 | 1880 | 240 |
| 200 | 86,2 | 45,3 | 4400 | 2870 | 350 |
| 220 | 89,2 | 67,9 | 7300 | 6460 | 420 |

Die Verwendung von in der Literatur beschriebenen Hydrierkatalysatoren (Pd, Ru, Rh, VB4-7) resultiert somit in etwas verbesserten L*-Werten verglichen mit unbehandeltem Gemisch, die b*-Werte bleiben allerdings schlecht und teilweise erhöhen sich die Chlorwerte drastisch.

### Beispiel 1:

Die Herstellung erfolgte nach der allgemeinen Vorschrift I.1 und I.2, wobei als Katalysator in I.1 ein Katalysator mit der Zusammensetzung 60% CuO, 10% Mn₂O₃ und 30% Aluminiumoxid eingesetzt wurde. Als MDA wurde MDA-1 eingesetzt. Die bei den jeweiligen Temperaturen erhaltenen Farbzahlen und Chlorwerte sind in der folgenden Tabelle angegeben:

| Hydriertemperatur [°C] | L* | b* | Totalchlor [ppm] | DHC [ppm] | EHC [ppm] |
|---|---|---|---|---|---|
| 100 | 78,2 | 45,9 | 2000 | 1490 | 240 |
| 120 | 85,0 | 39,9 | 1900 | 1430 | 250 |
| 140 | 88,0 | 32,4 | 2100 | 1480 | 180 |
| 160 | 91,9 | 25,4 | 1900 | 1740 | 190 |
| 180 | 94,3 | 25,1 | 2800 | 2430 | 190 |
| 200 | 95,7 | 16,6 | 4100 | 3370 | 180 |
| 220 | 94,9 | 24,9 | 7000 | 5440 | 230 |

### Beispiel 2:

Die Herstellung erfolgte nach der allgemeinen Vorschrift I.1 und 1.2, wobei als Katalysator in I.1 ein Katalysator mit der Zusammensetzung 57% CuO, 15% Cu, 24% Aluminiumoxid, 4% La₂O₃ eingesetzt wurde. Als MDA wurde MDA-1 eingesetzt. Die bei den jeweiligen Temperaturen erhaltenen Farbzahlen und Chlorwerte sind in der folgenden Tabelle angegeben:

| Hydriertemperatur [°C] | L* | b* | Totalchlor [ppm] | DHC [ppm] | EHC [ppm] |
|---|---|---|---|---|---|
| 160 | 90,4 | 33,9 | 3400 | 1110 | 180 |
| 180 | 96,9 | 22,8 | 3500 | 2400 | 190 |
| 200 | 97,1 | 18,8 | 4100 | 4110 | 180 |
| 220 | 95,7 | 22,9 | 7200 | 6230 | 200 |

### Beispiel 3:

Die Herstellung erfolgte nach der allgemeinen Vorschrift I.1 und 1.2, wobei als Katalysator in I.1 ein Katalysator mit der Zusammensetzung 57% CuO, 15% Cu, 24% Aluminiumoxid, 4% La₂O₃ eingesetzt wurde. Als MDA wurde MDA-3 eingesetzt. Dabei wurden bei einer Hydriertemperatur von 160°C nach Phosgenierung Farbzahlen von L* = 98,2 und b* = 11,3 erhalten. Es resultierten Chlorwerte von TC = 1900 ppm, DHC = 1250 ppm und EHC = 140 ppm.

### Beispiel 4:

Die Herstellung erfolgte nach der allgemeinen Vorschrift I.1 und 1.2, wobei als Katalysator in I.1 ein Katalysator mit der Zusammensetzung 25% CuO, 68% CuCrO₄, 7% BaO eingesetzt wurde. Als MDA wurde MDA-1 eingesetzt. Die bei den jeweiligen Temperaturen erhaltenen Farbzahlen und Chlorwerte sind in der Tabelle angegeben.

| Hydriertemperatur [°C] | L* | b* | Totalchlor [ppm] | DHC [ppm] | EHC [ppm] |
|---|---|---|---|---|---|
| 100 | 79,5 | 49,5 | 2000 | 1080 | 210 |
| 120 | 88,7 | 36,8 | 2100 | 1020 | 210 |
| 140 | 89,1 | 34,9 | 2200 | 1040 | 200 |
| 160 | 92,7 | 26,3 | 2500 | 1060 | 190 |
| 180 | 96,0 | 17,4 | 2600 | 1280 | 140 |
| 200 | 97,5 | 15,6 | 3100 | 1450 | 170 |
| 220 | 96,8 | 24,7 | 6400 | 4580 | 330 |

### Beispiel 5:

Die Herstellung erfolgte nach der allgemeinen Vorschrift I.1 und I.2, wobei als Katalysator in 1.1 ein Katalysator mit der Zusammensetzung 40% CuO und 40 % ZnO und 20% Aluminiumoxid eingesetzt wurde. Als MDA wurde MDA-1 eingesetzt. Die bei den jeweiligen Temperaturen erhaltenen Farbzahlen und Chlorwerte sind in der folgenden Tabelle angegeben.

| Hydriertemperatur [°C] | L* | b* | Totalchlor [ppm] | DHC [ppm] | EHC [ppm] |
|---|---|---|---|---|---|
| 100 | 85,2 | 38,7 | 2500 | 1170 | 260 |
| 120 | 94,8 | 19,9 | 2400 | 1150 | 200 |
| 140 | 97,5 | 15,4 | 2700 | 1300 | 240 |
| 160 | 97,0 | 16,2 | 3700 | 1570 | 200 |
| 180 | 97,2 | 25,3 | 4400 | 2510 | 240 |
| 200 | 98,0 | 18,8 | 5700 | 4010 | 280 |
| 220 | 98,2 | 17,0 | 11000 | 9000 | 300 |

### Beispiel 6:

Die Herstellung erfolgte nach der allgemeinen Vorschrift I.1 und 1.2, wobei als Katalysator in I.1 ein Katalysator mit der Zusammensetzung 25% CuO auf SiO₂ eingesetzt wurde. Als MDA wurde MDA-2 eingesetzt. Die bei den jeweiligen Temperaturen erhaltenen Farbzahlen und Chlorwerte sind in der Tabelle angegeben.

| Hydriertemperatur [°C] | L* | b* | Totalchlor [ppm] | DHC [ppm] | EHC [ppm] |
|---|---|---|---|---|---|
| 80 | 80,2 | 51,8 | 1800 | 1220 | 230 |
| 100 | 81,0 | 51,4 | 1700 | 1380 | 220 |
| 120 | 82,0 | 49,1 | 1900 | 1250 | 210 |
| 140 | 88,6 | 36,0 | 2400 | 1430 | 240 |
| 160 | 94,1 | 23,4 | 2600 | 1600 | 240 |
| 180 | 96,7 | 16,8 | 3100 | 1820 | 220 |
| 200 | 97,1 | 20,5 | 4000 | 2190 | 250 |

### Beispiel 7:

Die Herstellung erfolgte nach der allgemeinen Vorschrift I.1 und 1.2, wobei als Katalysator in I.1 ein Katalysator mit der Zusammensetzung 45% CuO , 20% MgO, 35 % SiO₂ eingesetzt wurde. Als MDA wurde MDA-2 eingesetzt. Die bei den jeweiligen Temperaturen erhaltenen Farbzahlen und Chlorwerte sind in der Tabelle angegeben

| Hydriertemperatur [°C] | L* | b* | Totalchlor [ppm] | DHC [ppm] | EHC [ppm] |
|---|---|---|---|---|---|
| 100 | 81,1 | 48,6 | 1900 | 910 | 230 |
| 120 | 89,1 | 33,6 | 1900 | 1030 | 230 |
| 140 | 90,8 | 25,2 | 1800 | 1290 | 180 |
| 160 | 91,0 | 28,3 | 2000 | 1370 | 170 |
| 180 | 97,3 | 16,2 | 2000 | 1410 | 140 |
| 200 | 96,8 | 20,6 | 3600 | 2670 | 160 |

### Beispiel 8:

Die Herstellung erfolgte nach der allgemeinen Vorschrift I.1 und 1.2, wobei als Katalysator in I.1 ein Katalysator mit der Zusammensetzung 61% CuO auf 39 % Al₂O₃ eingesetzt wurde. Als MDA wurde MDA-2 eingesetzt. Die bei den jeweiligen Temperaturen erhaltenen Farbzahlen und Chlorwerte sind in der Tabelle angegeben.

| Hydriertemperatur [°C] | L* | b* | Totalchlor [ppm] | DHC [ppm] | EHC [ppm] |
|---|---|---|---|---|---|
| 100 | 78,3 | 44,8 | 2800 | 960 | 260 |
| 120 | 83,0 | 37,6 | 1800 | 1050 | 230 |
| 140 | 89,1 | 29,5 | 1600 | 1160 | 250 |
| 160 | 95,0 | 19,0 | 1900 | 1440 | 180 |
| 180 | 96,9 | 17,2 | 2300 | 2180 | 170 |
| 200 | 98,0 | 15,5 | 3900 | 4330 | 200 |
| 220 | 96,9 | 49,6 | 5200 | 8570 | 310 |

### Beispiel 9:

Die Herstellung erfolgte nach der allgemeinen Vorschrift I.1 und I.2, wobei als Katalysator in I.1 ein Katalysator mit der Zusammensetzung 25% CuO, 73% CuCr₂O₄, 2% C eingesetzt wurde. Als MDA wurde MDA-2 eingesetzt. Die bei den jeweiligen Temperaturen erhaltenen Farbzahlen und Chlorwerte sind in der Tabelle angegeben.

| Hydriertemperatur [°C] | L* | b* | Totalchlor [ppm] | DHC [ppm] | EHC [ppm] |
|---|---|---|---|---|---|
| 120 | 83,9 | 42,7 | 2800 | 900 | 240 |
| 140 | 86,2 | 41,4 | 1500 | 960 | 180 |
| 160 | 89,0 | 25,3 | 1700 | 1070 | 170 |
| 180 | 93,6 | 25,7 | 2000 | 1430 | 180 |
| 200 | 90,8 | 44,2 | 3500 | 2270 | 150 |
| 220 | 94,6 | 41,1 | 5700 | 5870 | 250 |

### Beispiel 10:

Die Herstellung erfolgte nach der allgemeinen Vorschrift I.1 und 1.2, wobei als Katalysator in I.1 ein Katalysator mit der Zusammensetzung 19% CuO, 55% CuCr₂O₄, 12% BaCrO₄, 12% Na₂SiO₃, 2% C eingesetzt wurde. Als MDA wurde MDA-2 eingesetzt. Die bei den jeweiligen Temperaturen erhaltenen Farbzahlen und Chlorwerte sind in der Tabelle angegeben.

| Hydriertemperatur [°C] | L* | b* | Totalchlor [ppm] | DHC [ppm] | EHC [ppm] |
|---|---|---|---|---|---|
| 100 | 72,5 | 49,3 | 1400 | 870 | 230 |
| 120 | 75,3 | 44,4 | 1400 | 850 | 170 |
| 140 | 82,2 | 33,9 | 1500 | 950 | 200 |
| 160 | 83,8 | 29,1 | 1600 | 1030 | 200 |
| 180 | 92,4 | 19,8 | 1700 | 1230 | 150 |
| 200 | 96,7 | 17,8 | 2800 | 1790 | 140 |
| 220 | 96,6 | 24,9 | 3700 | 2900 | 130 |

### Beispiel 11:

Die Herstellung erfolgte nach der allgemeinen Vorschrift I.1 und I.2, wobei als Katalysator in I.1 ein Katalysator mit der Zusammensetzung 33% CuO, 40% Cr₂O₃, 16% CuCr₂O₄, 5% MnO₂, ,4% Na₂SiO₃, 2 % C eingesetzt wurde. Als MDA wurde MDA-2 eingesetzt. Die bei den jeweiligen Temperaturen erhaltenen Farbzahlen und Chlorwerte sind in der Tabelle angegeben.

| Hydriertemperatur [°C] | L* | b* | Totalchlor [ppm] | DHC [ppm] | EHC [ppm] |
|---|---|---|---|---|---|
| 100 | 75,5 | 44,4 | 3400 | 930 | 290 |
| 120 | 77,0 | 44,2 | 1400 | 870 | 210 |
| 140 | 86,6 | 34,6 | 1600 | 1040 | 220 |
| 160 | 81,1 | 28,2 | 2000 | 1130 | 140 |
| 180 | 96,5 | 19,2 | 2800 | 1910 | 180 |
| 200 | 96,4 | 21,4 | 3300 | 2840 | 220 |
| 220 | 96,4 | 23,0 | 5600 | 4600 | 220 |

### Beispiel 12:

In einen Reaktor wurden 40 ml eines Katalysators mit der Zusammensetzung 57% CuO, 15% Cu, 24% Aluminiumoxid, 4% La₂O₃ gefüllt und wie unter der allgemeinen Vorschrift I.1 aktiviert. Anschließend wurde MDA-1 mit 25 ml/h in Rieselfahrweise bei 150°C und 30 bar Druck über den Katalysator gefahren. Zusätzlich wurde Wasserstoff mit 10 NI/h zudosiert. Die Gesamtlaufzeit betrug 4200 Stunden, wobei in regelmäßigen Abständen Proben genommen wurden. Diese wurden am Rotationsverdampfer (1 mbar, 160°C, 2 h) von Wasser und Anilin befreit und nach allgemeiner Arbeitsvorschrift I.2 phosgeniert. Die Farbzahlen und Chlorwerte der phosgenierten Proben sind in folgender Tabelle aufgelistet:

| Laufzeit [h] | L* | b* | Totalchlor [ppm] | DHC [ppm] | EHC [ppm] |
|---|---|---|---|---|---|
| 240 | 94,8 | 20,7 | 1960 | 1320 | 170 |
| 1080 | 94,0 | 23,2 | 2100 | 1120 | 200 |
| 2360 | 95,3 | 21,0 | 2040 | 1240 | 170 |
| 3540 | 93,3 | 25,2 | 2100 | 1030 | 180 |
| 4200 | 93,5 | 24,8 | 2050 | 1010 | 200 |

Die Verwendung der erfindungsgemäßen Kupferkatalysatoren resultiert, wie die Beispiele zeigen, insbesondere bei den oben genannten bevorzugten Hydriertemperaturen in den bevorzugten Farbzahlen, die größtenteils bei Werten von L* > 90 und b* < 40 liegen, wobei gleichzeitig auch die Chlorwerte in den gewünschten Bereichen liegen und der Totalchlorgehalt TC größtenteils bei < 6000 ppm und der DHC größtenteils bei < 5000 ppm liegen.

## Patentansprüche

1. Verfahren zur Herstellung eines Isocyants, umfassend
(i) Hydrieren eines ein Amin enthaltenden Gemischs (Gi) in Anwesenheit eines Kupfer enthaltenden Hydrierkatalysator unter Erhalt eines das Amin enthaltenden Gemischs (Gii);
(ii) Umsetzen des Gemischs (Gii) mit Phosgen unter Erhalt eines das Isocyanat enthaltenden Gemischs (Giii).

2. Verfahren gemäß Anspruch 1, wobei der Hydrierkatalysator Kupfer in einer Menge im Bereich von 0,1 bis 100 Gew.-%. bevorzugt im Bereich von 10 bis 60 Gew.-%, bezogen auf das Gesamtgewicht des Katalysators und berechnet als Metall, enthält.

3. Verfahren gemäß Anspruch 1 oder 2, wobei der Hydrierkatalysator ein Trägermaterial, ausgewählt aus der Gruppe bestehend aus Siliziumdioxid, Aluminiumoxid, Zinkoxid, Titandioxid, Manganoxid, Zirkonoxid, Lanthanoxid und einer Mischung aus zwei oder mehr dieser Materialien, enthält.

4. Verfahren gemäß Anspruch 3, wobei der Hydrierkatalysator das Trägermaterial in einer Menge im Bereich von 40 bis 90 Gew.-%, bezogen auf das Gesamtgewicht des Hydrierkatalysators, enthält.

5. Verfahren gemäß einem der Ansprüche 1 bis 4, wobei der Hydrierkatalysator, zusätzlich zu Kupfer und gegebenenfalls Trägermaterial, mindestens ein Element ausgewählt aus der Gruppe bestehend aus Lanthan, Magnesium, Mangan, Barium, Kohlenstoff, Chrom, Silber, Zink, Natrium, Gold und einer Mischung aus zwei oder mehr dieser Elemente enthält.

6. Verfahren gemäß einem der Ansprüche 1 bis 5, wobei das Amin ein Gemisch aus mindestens zwei Diaminen der Diphenylmethan-Reihe und mindestens zwei Polyaminen der Diphenylmethan-Reihe ist und das Isocyanat ein Gemisch aus mindestens zwei Diisocyanaten der Diphenylmethan-Reihe und mindestens zwei Polyisocyanaten der Diphenylmethan-Reihe ist.

7. Verfahren gemäß einem der Ansprüche 1 bis 6, wobei das Amin durch Umsetzen von Anilin und Formaldehyd in Gegenwart eines sauren Katalysators hergestellt wird.

8. Verfahren gemäß einem der Ansprüche 1 bis 7, wobei das Gemisch (Gi) zusätzlich Anilin oder zusätzlich Wasser oder zusätzlich Anilin und Wasser enthält.

9. Verfahren gemäß Anspruch 8, wobei das Gemisch (Gi) Anilin in einer Menge im Bereich von bis zu 75 Gew.-%, bevorzugt von bis zu 50 Gew.-% und besonders bevorzugt von bis zu 40 Gew.-% enthält.

10. Verfahren gemäß Anspruch 8 oder 9, wobei das Gemisch (Gi) Wasser in einer Menge im Bereich von bis zu 50 Gew.-%, bevorzugt im Bereich von bis zu 25 Gew.-%, besonders bevorzugt im Bereich von bis zu 10 Gew.-%, bezogen auf das Gesamtgewicht des Gemisches (Gi) enthält.

11. Verfahren gemäß einem der Ansprüche 1 bis 10 wobei das Hydrieren gemäß (i) bei einer Temperatur im Bereich von 20 bis 300 °C, bevorzugt 70 bis 200 °C, weiter bevorzugt von 100 bis 220 °C, insbesondere bevorzugt von 120 bis 200 °C und bei einem Druck im Bereich von 1 bis 300 bar, bevorzugt 10 bis 100 bar und besonders bevorzugt 20 bis 50 bar erfolgt.

12. Verfahren gemäß einem der Ansprüche 1 bis 11, wobei das Umsetzen gemäß (ii) bei einer Temperatur im Bereich von 100 bis 350 °C und bei einem Druck im Bereich von 0,5 bis 150 bar erfolgt.

13. Verfahren gemäß einem der Ansprüche 1 bis 12, wobei das Gemisch (Gi) zusätzlich mindestens eine Verbindung ausgewählt aus der Gruppe bestehend aus N-formylierten Diaminen der Diphenylmethan-Reihe, N-formylierten Polyaminen der Diphenylmethan-Reihe, und 3,4-Dihydrochinazolinen enthält.

14. Verfahren gemäß einem der Ansprüche 1 bis 13, wobei das Umsetzen gemäß (ii) in Gegenwart eines Lösungsmittels, bevorzugt in Gegenwart eines Lösungsmittels ausgewählt aus der Gruppe bestehend aus Chlorbenzolen und Kohlenwasserstoffen durchgeführt wird.

## Claims

1. A process for preparing an isocyanate, comprising
(i) hydrogenating a mixture (Gi) comprising an amine in the presence of a hydrogenation catalyst comprising copper to obtain a mixture (Gii) comprising the amine;
(ii)reacting the mixture (Gii) with phosgene to obtain a mixture (Giii) comprising the isocyanate.

2. The process according to claim 1, wherein the hydrogenation catalyst comprises copper in an amount in the range from 0.1 to 100% by weight, preferably in the range from 10 to 60% by weight, based on the total weight of the catalyst and calculated as the metal.

3. The process according to claim 1 or 2, wherein the hydrogenation catalyst comprises a support material selected from the group consisting of silicon dioxide, aluminium oxide, zinc oxide, titanium dioxide, manganese oxide, zirconium oxide, lanthanum oxide and a mixture of two or more of these materials.

4. The process according to claim 3, wherein the hydrogenation catalyst comprises the support material in an amount in the range from 40 to 90% by weight, based on the total weight of the hydrogenation catalyst.

5. The process according to any one of claims 1 to 4, wherein the hydrogenation catalyst, in addition to copper and any support material, comprises at least one element selected from the group consisting of lanthanum, magnesium, manganese, barium, carbon, chromium, silver, zinc, sodium, gold and a mixture of two or more of these elements.

6. The process according to any one of claims 1 to 5, wherein the amine is a mixture of at least two diamines of the diphenylmethane series and at least two polyamines of the diphenylmethane series, and the isocyanate is a mixture of at least two diisocyanates of the diphenylmethane series and at least two polyisocyanates of the diphenylmethane series.

7. The process according to any one of claims 1 to 6, wherein the amine is prepared by reacting aniline and formaldehyde in the presence of an acidic catalyst.

8. The process according to any one of claims 1 to 7, wherein the mixture (Gi) comprises additionally aniline or additionally water or additionally aniline and water.

9. The process according to claim 8, wherein the mixture (Gi) comprises aniline in an amount in the range of up to 75% by weight, preferably of up to 50% by weight and more preferably of up to 40% by weight.

10. The process according to claim 8 or 9, wherein the mixture (Gi) comprises water in an amount in the range of up to 50% by weight, preferably in the range of up to 25% by weight, more preferably in the range of up to 10% by weight, based on the total weight of the mixture (Gi).

11. The process according to any one of claims 1 to 10, wherein the hydrogenation in (i) is effected at a temperature in the range from 20 to 300°C, preferably 70 to 200°C, more preferably from 100 to 220°C, especially preferably from 120 to 200°C, and a pressure in the range from 1 to 300 bar, preferably 10 to 100 bar and more preferably 20 to 50 bar.

12. The process according to any one of claims 1 to 11, wherein the reaction in (ii) is effected at a temperature in the range from 100 to 350°C and at a pressure in the range from 0.5 to 150 bar.

13. The process according to any one of claims 1 to 12, wherein the mixture (Gi) additionally comprises at least one compound selected from the group consisting of N-formylated diamines of the diphenylmethane series, N-formylated polyamines of the diphenylmethane series, and 3,4-dihydroquinazolines.

14. The process according to any one of claims 1 to 13, wherein the reaction in (ii) is performed in the presence of a solvent, preferably in the presence of a solvent selected from the group consisting of chlorobenzenes and hydrocarbons.

## Revendications

1. Procédé pour la préparation d'un isocyanate, comprenant
(i) l'hydrogénation d'un mélange contenant une amine (Gi) en présence d'un catalyseur d'hydrogénation contenant du cuivre avec obtention d'un mélange contenant l'amine (Gii) ;
(ii) transformation du mélange (Gii) avec du phosgène avec obtention d'un mélange contenant l'isocyanate (Giii).

2. Procédé selon la revendication 1, le catalyseur d'hydrogénation contenant le cuivre en une quantité dans la plage de 0,1 à 100% en poids, de préférence dans la plage de 10 à 60% en poids, par rapport au poids total du catalyseur et calculé sous forme de métal.

3. Procédé selon la revendication 1 ou 2, le catalyseur d'hydrogénation contenant un matériau support, choisi dans le groupe constitué par le dioxyde de silicium, l'oxyde d'aluminium, l'oxyde de zinc, le dioxyde de titane, l'oxyde de manganèse, l'oxyde de zirconium, l'oxyde de lanthane et un mélange de deux de ces matériaux ou plus.

4. Procédé selon la revendication 3, le catalyseur d'hydrogénation contenant le matériau support en une quantité dans la plage de 40 à 90% en poids, par rapport au poids total du catalyseur d'hydrogénation.

5. Procédé selon l'une quelconque des revendications 1 à 4, le catalyseur d'hydrogénation contenant, en plus du cuivre et le cas échéant du matériau support, au moins un élément choisi dans le groupe constitué par le lanthane, le magnésium, le manganèse, le baryum, le carbone, le chrome, l'argent, le zinc, le sodium, l'or et un mélange de deux de ces éléments ou plus.

6. Procédé selon l'une quelconque des revendications 1 à 5, l'amine étant un mélange d'au moins deux diamines de la série des diphénylméthanes et d'au moins deux polyamines de la série des diphénylméthanes et l'isocyanate étant un mélange d'au moins deux diisocyanates de la série des diphénylméthanes et au moins deux polyisocyanates de la série des diphénylméthanes.

7. Procédé selon l'une quelconque des revendications 1 à 6, l'amine étant préparée par la transformation d'aniline et de formaldéhyde en présence d'un catalyseur acide.

8. Procédé selon l'une quelconque des revendications 1 à 7, le mélange (Gi) contenant en plus de l'aniline ou en plus de l'eau ou en plus de l'aniline et de l'eau.

9. Procédé selon la revendication 8, le mélange (Gi) contenant de l'aniline en une quantité dans la plage de jusqu'à 75% en poids, de préférence de jusqu'à 50% en poids et de manière particulièrement préférée de jusqu'à 40% en poids.

10. Procédé selon la revendication 8 ou 9, le mélange (Gi) contenant de l'eau en une quantité dans la plage de jusqu'à 50% en poids, de préférence dans la plage de jusqu'à 25% en poids, de manière particulièrement préférée dans la plage de jusqu'à 10% en poids, par rapport au poids total du mélange (Gi).

11. Procédé selon l'une quelconque des revendications 1 à 10, l'hydrogénation selon (i) ayant lieu à une température dans la plage de 20 à 300°C, de préférence de 70 à 200°C, plus préférablement de 100 à 220°C, en particulier de préférence de 120 à 200°C et à une pression dans la plage de 1 à 300 bars, de préférence de 10 à 100 bars et de manière particulièrement préférée de 20 à 50 bars.

12. Procédé selon l'une quelconque des revendications 1 à 11, la transformation selon (ii) ayant lieu à une température dans la plage de 100 à 350°C et à une pression dans la plage de 0,5 à 150 bars.

13. Procédé selon l'une quelconque des revendications 1 à 12, le mélange (Gi) contenant en outre au moins un composé choisi dans le groupe constitué par les diamines N-formylées de la série des diphénylméthanes, les polyamines N-formylées de la série des diphénylméthanes et les 3,4-dihydroquinazolines.

14. Procédé selon l'une quelconque des revendications 1 à 13, la transformation selon (ii) ayant lieu en présence d'un solvant, de préférence en présence d'un solvant choisi dans le groupe constitué par les chlorobenzènes et les hydrocarbures.
